(19) Europäisches Patentamt / European Patent Office / Office européen des brevets

(11) **EP 3 850 640 B1**

(12) **EUROPÄISCHE PATENTSCHRIFT**

(45) Veröffentlichungstag und Bekanntmachung des Hinweises auf die Patenterteilung:
**05.07.2023   Patentblatt 2023/27**

(21) Anmeldenummer: **19789857.0**

(22) Anmeldetag: **10.09.2019**

(51) Internationale Patentklassifikation (IPC):
***G16H 50/20*** *(2018.01)*

(52) Gemeinsame Patentklassifikation (CPC):
**G16H 50/20**

(86) Internationale Anmeldenummer:
**PCT/DE2019/100808**

(87) Internationale Veröffentlichungsnummer:
**WO 2020/052713 (19.03.2020 Gazette 2020/12)**

(54) **VERFAHREN UND EINRICHTUNG ZUR HERZÜBERWACHUNG**

METHOD AND APPARATUS FOR CARDIAC MONITORING

PROCEDE ET APPAREIL DE SURVEILLANCE CARDIAQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.09.2018   DE 102018121974**

(43) Veröffentlichungstag der Anmeldung:
**21.07.2021   Patentblatt 2021/29**

(73) Patentinhaber: **Cardisio GmbH**
**60549 Frankfurt (DE)**

(72) Erfinder:
• **BAUMEISTER, Meik**
**79206 Breisach (DE)**
• **TENDERICH, Gero**
**47918 Tönisvorst (DE)**

(74) Vertreter: **Dammertz, Ulrich**
**Patentanwaltskanzlei Dammertz**
**Ferdinand Strasse 10**
**47906 Kempen (DE)**

(56) Entgegenhaltungen:
EP-A2- 0 086 429        WO-A1-03/057031
CN-A- 108 420 454       DE-A1-102012 106 893
JP-A- 2008 220 556

# EP 3 850 640 B1

**Beschreibung**

Die Erfindung betrifft ein Verfahren zur Früherkennung des Vorliegens einer koronaren Herzkrankheit oder einer Herzrhythmusstörung eines zu untersuchenden Patienten nach dem Oberbegriff von Anspruch 1, eine solche Einrichtung nach dem Oberbegriff von Anspruch 10.

Zu den häufigsten Erkrankungen von Patienten zählt die Koronare Herzkrankheit, kurz KHK. Die derzeit hierzu vorliegende Prävalenz in Deutschland (bei Männern ca. 30%, bei Frauen ca. 15%) belegt einen großen Bedarf an Geräten, mit denen solche Probleme schnell erkannt und die Menschen bzw. Patienten einer frühen Therapie oder Beratung zugeführt werden können. Ein weiteres großes Feld der Störungen in der Arbeit des Herzens betrifft die Arrhythmie oder Herzrhythmusstörungen. Das Herz eines Menschen besitzt ein kompliziertes Erregungsbildungs- und Leitsystem (Erregungssystem), welches der Treiber der Herzbewegungen ist. Durch die Teilung des Herzens in die Kammern und Vorhöfe ist es notwendig, den Blutfluss durch Muskelkontraktionen zu regulieren. Im Zusammenhang mit der rhythmischen Kontraktion des Herzmuskels besteht das Erregungssystem aus speziellen Muskelzellen, welche in der Lage sind, sich spontan selbst zu de- und repolarisieren. Damit kann ein Rhythmus, der in erster Linie vom Sinusknoten generiert wird, aufrechterhalten werden. Bei einer Störung dieses Erregungssystem kommt es zu Beeinträchtigungen der Pumpleistung des Herzens und somit zu Versorgungsengpässen. Nicht alle Symptome sind lebensgefährlich, können aber, sobald sie häufig auftreten, gute Indikatoren für drohende lebensgefährliche Zustände werden.

Der Referenzstandard zur Erkennung einer KHK ist die Koronarangiographie, allerdings wird hiermit der Bereich der Früherkennung der KHK verlassen. Hierbei wird ein Katheter in das Herz eingeführt, um u.a. eine Druckmessung der Aorta durchzuführen. Weiterhin werden Kontrastmittel verwendet, die die Darstellung der Koronararterien des Herzens erlauben. Entsprechend handelt es sich um eine invasive Methode mit den damit verbundenen gesundheitlichen Risiken. Alternativen dazu sind eine Angiographie mittels CT und MRT. Bei diesen Alternativen werden ebenfalls Kontrastmittel verwendet, mit den damit verbundenen gesundheitlichen Risiken. Diese Verfahren sind nachteilig mit einem hohen Aufwand und großen Kosten verbunden.

Gemessen am goldenen Standard der Koronardiagnostik der Koronarangiographie mit einer Spezifität und Sensitivität von 100 %, zeigen nicht invasive Verfahren wie das Ruhe-EKG und die Ruhe-Echokardiographie eine deutlich geringere Sensitivität und Spezifität (von weniger als 30% bzw. 70%). Erst unter Belastung erhöhen sich diese für beide Verfahren, machen aber jeweils die Anwesenheit eines Arztes notwendig. Ferner sind die Ergebnisse im Fall der Stressechokardiographie zusätzlich Untersucher abhängig. Hinzu kommt, dass beide dieser Verfahren zusätzlich einen Zeitraum von mindestens 15-30 Minuten benötigen. Weitere nicht invasive Verfahren wie Myokard-perfusionssyntigraphie (MPS), Koronarcomputer-Tomographie (CCT) oder kardiale Magnetresonanztomographie (MRT) erfordern einen erheblichen apparativen sowie personellen Aufwand, die Anwesenheit eines Arztes und stellen ferner einen erheblichen Kostenfaktor dar.

Für eine nicht-invasive Untersuchung von Patienten kann eine EKG- Untersuchung durchgeführt werden. Auf diesem Gebiet bieten moderne Softwaresysteme einem Arzt viele Werkzeuge an, um eine Vermessung von Patienten zumindest schnell durchzuführen. Gleichwohl ist die Auswertung eines EKG maßgeblich von der Erfahrung des behandelnden Arztes abhängig und damit unsicher. Weiterhin kann eine Überlastung von Ärzten zu Fehldiagnosen führen. Zur Lösung dieser Problematik haben sich im Bereich der EKG-Analyse automatische Vermessungssysteme etabliert. Allerdings beschränken sich diese automatischen Systeme auf die Vermessung von EKGs und bilden somit die Erfahrung beziehungsweise definierte Parameter (bspw. QT-Zeit oder ST-Hebung) in der Software ab. Es ist somit nur die Unsicherheit durch den Faktor Mensch entfernt worden.

Die Parameter eines EKG, vor allem in Ruhe, sind zur Erkennung von KHK in der Regel unzureichend. Aus diesem Grund wird mit Patienten ein Belastungs-EKG durchgeführt, um damit die Früherkennung einer KHK zu ermöglichen. Dafür ist es erforderlich, dass der Patient einer körperlichen Belastung ausgesetzt wird. Es erfolgt somit eine Provokation der Symptome, die zum Herzinfarkt führen können. Dies macht bei einer solchen Messung stets die Anwesenheit eines Arztes notwendig. Der Nachteil einer solchen Vorgehensweise liegt in den damit verbundenen Risiken. Abgesehen von der Gefahr für den Patienten ist es nicht für alle Menschen möglich solche Tests durchzuführen. Vor allem in den Risikogruppen (Diabetes, Übergewicht, hohes Alter) sind die Menschen nicht immer in der Lage, die notwendige Belastung überhaupt zu erreichen, mangels ausreichender Kondition. Es gibt als Alternative die Belastung durch Medikamente und dann Untersuchung mittels Echokardiographie (Stress-Echo) mittels Ultraschall. Allerdings ist das Echo eher zur Untersuchung von Bewegungsstörungen geeignet und bietet ebenfalls nicht die Sicherheit einer automatischen Auswertung.

Mit derzeit verfügbaren EKG-Geräte ist es nicht möglich, frühzeitig Anzeichen von KHK zu erkennen oder dem Arzt in geeigneter Form darzustellen. Allen bislang bekannten Geräten ist gemein, dass sie mehrere Ableitungen gleichzeitig darstellen, mit zumeist 3 bis 12 Kanälen. Die Software zur Darstellung erlaubt in der Regel die Konfiguration der Ansicht, welche es einem Arzt erlaubt, sich auf einzelne Ableitungen zu konzentrieren und entsprechend bekannter Richtlinien auszuwerten. Der Verdacht auf einen drohenden Herzinfarkt (das Ergebnis einer unbehandelten KHK) entsteht u.a. bei ST-Streckenhebungen >= 0,1 mV in mindestens zwei Extremitätenableitungen und >= 0,2 mV in zwei benachbarten Brustwandableitungen. Allerdings bedeutet das Fehlen dieser Hebung nicht, dass ein Herzinfarkt ausgeschlossen ist, denn der überwiegende Teil der Herzinfarkte sind Nicht-Hebungsinfarkte (NSTEMI).

Aus EP 86 429 B1 ist ein Verfahren zur Kardiogoniometrie bekannt, dass zur Klasse der Vektor-kardiometrischen Methoden gehört. Dieses Verfahren erlaubt eine sehr genaue Ermittlung des das elektrische Feld des Herzens abbildenden Summenvektors und stellt ihn in einem kartesischen Koordinatensystem dar, das an der Herzlängsachse (anstatt an der Körperachse) ausgerichtet ist. Hierbei werden für die Diagnose von Herzkrankheiten die Potentiale, die während einer Depolarisierung und Repolarisierung (R- und T-Schleife) des Herzmuskels maximal erreicht werden, verwendet sowie die Richtungen der entsprechenden zwei potentialmässig ausgezeichneten Vektoren und der Raumwinkel zwischen den beiden ausgezeichneten Vektoren. Dies bedeutet, dass für diagnostische Zwecke bezüglich des Betrags ausgezeichnete Vektoren und getrennt davon deren Richtungen und einzelne Komponenten in kartesischen Koordinaten, Kugelkoordinaten und Zylinderkoordinaten, parametrisiert werden.

Die Ableitungspunkte der Kardiogoniometrie gemäß EP 86 429 B1, zwischen denen Potentialdifferenzen gemessen werden, sind:

- E1 entsprechend dem Punkt für die Spannung V4 (gemäß Wilson),
- E2 sagittal zu E1 und entsprechend dem Punkt für die Spannung V8 (gemäß Wilson),
- E3 lotrecht zur Strecke E1 -E2, über E1 in einem Abstand von 0,7-mal dem Abstand zwischen E1 und E2,
- E4 waagrecht von E3 nach der rechten Patientenseite in einem Abstand von 0,7-mal dem Abstand zwischen E1 und E2.

Eine Vektoraddition der gemessenen Potentialdifferenzen ergibt einen Raumvektor V bzw. Projektionen des Raumvektors V auf eine x, y und z-Achse, wobei die x-Achse parallel zur Strecke E1-E2, und die y-Achse parallel zur Strecke E1-E4 und wobei die z-Achse senkrecht auf der durch E1, E2 und E4 aufgespannten Ebene (schrägsagittale Ebene) steht.

Die elektrische Herzaktion erzeugt ein sich über die Zeit änderndes elektrisches Feld. Der gemäß EP 86 429 B1 ermittelte Raumvektor V ist eine Näherung an den tatsächlichen räumlichen Summenvektor dieses Feldes. Dabei entspricht die Richtung des Raumvektors der Feldrichtung und die Länge (Betrag oder Potential) des Raumvektors der Feldstärke. Die drei Spannungen X, Y und Z [mV] stellen die Teilvektoren oder Komponenten des Raumvektors V in einem dreidimensionalen kartesischen Koordinatensystem dar, vorzugsweise den nach der kardiogoniometrischen Methode ermittelten Raumvektor im oben genannten Koordinatensystem. Das Potential berechnet sich als Quadratwurzel aus $(X^2+Y^2+Z^2)$.

Aus WO 99/36860 ist bekannt, dass der zeitliche Verlauf des Betrags des Raumvektors V durch Bestimmung von Maxima und Minima (Nullstellen im Verlauf des Differentialquotienten) in die Bereiche R+, R-, ST, T+ und T- aufgeteilt wird. In diesen Bereichen werden die periodisch registrierten Vektorbeträge aufsummiert und die solcher Art ermittelten Integralwerte für diagnostische Zwecke weiterverwendet. Somit werden schlingenweise Summen von Vektorbeträgen parametrisiert.

Des Weiteren ist aus WO 03/057031 A1 ein Verfahren zur Erzeugung von kardiometrischen Parametern bekannt, die insbesondere für Diagnosezwecke bei der Untersuchung des menschlichen Herzens verwendet werden können. Ein solches Verfahren ist in der WO 03/057031 A1 als "Vektor-Kardiometrie" bezeichnet und dient zur Erzeugung von kardiometrischen Parametern aus einem Vektor, der das elektrische Feld des Herzens abbildet und der z.B. nach bekannten Vektor-kardiometrischen Methoden ermittelt wird. Der Raumvektor wird von einem Koordinatenursprung ausgehend dargestellt, wobei eine von der Vektorspitze beschriebene räumliche Bahn und/oder eine räumliche Geschwindigkeit der sich entlang der Bahn bewegenden Vektorspitze im virtuellen Raum um den Koordinatenursprung parametrisiert wird. Die hierbei erzeugten Parameter bzw. die genannten Abweichungen sind insbesondere für die Diagnose von Herzkrankheiten oder -störungen geeignet.

Die vorstehend erläuterten Verfahren zur Ermittlung und Darstellung eines auf das Herz bezogenen Raumvektors nach der Kardiogoniometrie insbesondere gemäß EP 86 429 B1 unterliegen dem Nachteil, dass Elektroden bzw. Sensoren am menschlichen Körper in exakter Übereinstimmung zu den vier Ableitungspunkten E1-E4, an denen Potentialdifferenzen gemessen werden, anzubringen sind. Eine nicht korrekte Position einer Elektrode insbesondere am Ableitungspunkt E1 führt zu einer Verfälschung der Messwerte, so dass die hieraus gewonnene Analyse unbrauchbar und eine zuverlässige Diagnose für ein untersuchtes Herz hiermit nicht möglich ist.

Ein weiterer Nachteil bei den Technologien der vorstehend genannten Druckschriften zum Stand der Technik besteht darin, dass eine automatische Analyse in Bezug auf die von einem Patienten gewonnenen dreidimensionalen Signale hierbei nicht vorgesehen ist.

Entsprechend liegt der Erfindung die Aufgabe zugrunde, eine Technologie auf dem Gebiet der nicht-invasiven Überwachung des Herzens zu schaffen, mit der in kürzerer Zeit eine verbesserte Untersuchung von Patienten möglich ist und insbesondere auch automatische Analysen möglich sind.

Die obige Aufgabe wird durch ein jeweiliges Verfahren mit den in Anspruch 1, Anspruch 14 und Anspruch 15 angegebenen Merkmalen, und durch eine Einrichtung mit den Merkmalen von Anspruch 10 gelöst. Vorteilhafte Weiterbildungen der Erfindung sind in den abhängigen Ansprüchen definiert.

Ein Verfahren nach der vorliegenden Erfindung dient zur Früherkennung des Vorliegens einer koronaren Herzkrankheit (KHK) und/oder einer Herzrhythmusstörung (HRS) und/oder einer Herzklappeninsuffizienz eines zu untersuchenden Patienten, und umfasst die Schritte:

(i) nicht-invasive Aufnahme von EKG-Signalen am Herzen des Patienten in dessen Ruhezustand,
(ii) filtertechnische Aufbereitung der aufgenommenen EKG-Signale,
(iii) Überführung der gefilterten EKG-Signale in orthogonalisierte Messgrößen auf Grundlage der Vektorkardiographie, und
(iv) Eingabe der orthogonalisierten Messgrößen in ein System basierend auf Künstlicher Intelligenz (KI), in dem diese eine Gewichtung erfahren, die über bekannte Befunddaten von Vergleichspatienten über ein Training eines Neuronalen Netzes festgelegt wurde. Die orthogonalisierten Befunddaten werden auf einen Wert zwischen -1 und 1 abgebildet und lassen eine Diagnose des Patienten zu.

In gleicher Weise sieht die Erfindung auch eine Einrichtung zur Früherkennung des Vorliegens einer KHK und/oder einer HRS und/oder einer und/oder einer Herzklappeninsuffizienz eines zu untersuchenden Patienten, umfassend eine Mehrzahl von Sensoren, die am Körper des zu untersuchenden Patienten an vorbestimmten Ableitungspunkten positionierbar sind, um damit nicht-invasiv EKG-Signale am Herzen des Patienten vorzugsweise in dessen Ruhezustand aufzunehmen, mindestens einen Filter, mit dem die aufgenommenen EKG-Signale filterbar sind, eine Auswerteeinrichtung, mit der die gefilterten EKG-Signale auf Grundlage der Vektorkardiographie in orthogonalisierte Messgrößen überführbar sind, und ein System basierend auf Künstlicher Intelligenz (KI), das mit Befunddaten von Vergleichspatienten trainiert wurde, wobei die orthogonalisierten Messgrößen in das KI-System eingegeben und entsprechend der Gewichtung des Neuronalen Netzes auf Werte zwischen -1 und 1 funktional abgebildet werden, um damit eine Diagnose für den untersuchten Patienten zu erstellen.

Wie vorstehend bereits erläutert, wird an dieser Stelle nochmals gesondert darauf hingewiesen, dass das genannte Verfahren und die Einrichtung gemäß der vorliegenden Erfindung auch zur Früherkennung des Vorliegens einer Herzklappeninsuffizienz eines Patienten geeignet sind. Diesbezüglich versteht sich, dass dann die Befunddaten der Vergleichspatienten innerhalb des KI-Systems hieran angepasst sind.

Die theoretische Grundlage für das Analyseprinzip nach der vorliegenden Erfindung bildet die sogenannte "Vektorkardiographie", die im Bereich der kardialen Ischämie- Diagnostik als nicht-invasive Methode einen nennenswerten Beitrag leistet.

Der Erfindung nach dem vorstehend genannten Verfahren und der vorstehend genannten Einrichtung liegt die wesentliche Erkenntnis zugrunde, dass die Darstellung der elektrophysiologischen Eigenschaften des Herzens im Vergleich zur komplizierten Darstellung des oft verwendeten herkömmlichen EKG mit seinen bis zu 12 Ableitungen dadurch vereinfacht wird, dass die gefilterten EKG-Signale auf Grundlage der Vektorkardiographie zunächst in orthogonalisierte Messgrößen überführt werden. Hierbei werden die kardialen Potentiale in einem orthogonalen System gemessen und vektoriell summiert. Bei einer solchen dreidimensionalen Messung und Ortung des Herzpotentials können eine Vielzahl von neuen Parametern geschaffen bzw. berücksichtigt werden, was u.a. die Erkennung der Myokardischämie erlaubt.

Ein wesentlicher Vorteil der Erfindung besteht darin, dass damit eine automatische Analyse unter Verwendung von dreidimensionalen Signalen durchgeführt wird bzw. möglich ist. Hierdurch ist nicht nur eine profunde Analysegrundlage gewährleistet, sondern auch eine grafische Aufbereitung der elektrophysiologischen Eigenschaften des Herzens möglich. Damit wird im Vergleich zu den bislang eingesetzten EKG- Messungen und den dabei verwendeten bis zu 12 Ableitungen (= 12 Dimension) die ansonsten komplizierte Darstellung stark vereinfacht und auf eine intiutive Weise visualisiert, wodurch in der Analyse und Auswertung mit der vorliegenden Erfindung weniger Fehler auftreten.

Zweckmäßigerweise erfolgt die Messung bzw. die nicht-invasive Aufnahme von EKG-Signalen am Herzen des Patienten in dessen Ruhezustand (d.h. ohne körperliche Belastung) unter Verwendung von vier Oberflächenelektroden. Dies bedeutet, dass die EKG-Signale an insgesamt vier Ableitungspunkten am Körper des Patienten in Nähe des Herzens aufgenommen werden. Für diese Messung können folgende Darstellungsformen vorgesehen sein:

- Analoge orthogonale Projektionen X, Y, Z zur Kontrolle der technischen Qualität der Messung;
- Potential, gemessen in den verschiedenen Zeitabschnitten des Herzzyklus;
- Schlingen in 2D und 3D, zur Ortung des Potentials im Herzen,
- Maximalvektoren der Vorhof- und Ventrikel-Depolarisation und der Ventrikel-Repolarisation.

Ein zentraler Aspekt für die vorliegende Erfindung besteht darin, dass die orthogonalisierten Messgrößen in ein System basierend auf Künstlicher Intelligenz (KI), vorzugsweise in Form eines neuronalen Netzes oder einer Mehrzahl von solchen neuronalen Netzen, eingegeben werden, wobei dieses KI-System (bzw. neuronales Netz) mit bereits bekannten Befunden trainiert wurde und insoweit diese Befunddaten von Vergleichspatienten in dem KI-System gespeichert sind. Durch eine funktionale Abbildung der eingegebenen orthogonalisierten Messgrößen über ein neuronales Netzwerkes auf Werte zwischen -1 und 1 wird eine Diagnose für den untersuchten Patienten erstellt, wobei dieses KI-System mit Hilfe von Befunddaten von Vergleichspatienten trainiert wurde, welches typische Charakteristika der Messgrössen entsprechend deren Relevanz für den Gesundheitszustand gewichtet.

Bezüglich der bekannten Befunddaten von Vergleichspatienten, auf welchen das KI-System bzw. neuronale Netz basiert, wird gesondert hervorgehoben, dass für diese Vergleichspatienten eine eindeutige Kenntnis in Bezug auf deren Gesundheitszustand ("gesund" oder "krank") vorliegt. Diese Information kann z.B. mittels einer Koronarangiografie erhalten worden sein, die eine eindeutige Diagnose für die Vergleichspatienten ("gesund" oder "krank") liefert, oder aus anderen Quellen vorliegen. Auf Grundlage dessen erfolgt eine Klassifizierung und ein Clustering der Befunddaten (Diagnose als "gesund" oder "krank").

In vorteilhafter Weiterbildung der Erfindung werden die Ableitungen zwischen den jeweiligen vier Ableitungspunkten, an denen die EKG-Signale aufgenommen werden, sowohl in kartesischen Koordinaten als auch in Kugel- und Zylinderkoordinaten betrachtet. Eine solche Transformation in Kugelkoordinaten ermöglicht die Bestimmung der tatsächlichen Zeitpunkte der physiologischen Extrempunkte (d.h. der genaue Punkt des R-Peaks bzw. der T-Welle) während eines Herzschlags.

In vorteilhafter Weiterbildung des erfindungsgemäßen Verfahrens wird das KI-System bzw. das neuronale Netz vor dem Schritt (iv) trainiert. Dies bedeutet, dass das KI-System bzw. das neuronale Netz durch Eingabe von spezifischen Lernwerten trainiert bzw. optimiert werden, wobei die Anzahl dieser spezifischen Lernwerte etwa zwischen 10 und 30 liegen kann und z.B. 20 beträgt, jedenfalls deutlich geringer als 100 ist. In diesem Zusammenhang ist hervorzuheben, dass ein solches Training mittels der genannten spezifischen Lernwerte nicht während der eigentlichen Diagnose, sondern im Vorfeld durchgeführt wird, damit die im Trainingsset verfügbaren Daten Anwendung finden können.

Im Zusammenhang mit der Durchführung des Verfahrens und/oder dem Einsatz der Einrichtung nach der vorliegenden Erfindung wird darauf hingewiesen, dass praktisch jede untersuchte Person bzw. jeder Anwender, zu der bzw. dem eine eindeutige Kenntnis/Information bezüglich des Gesundheitszustands ("gesund" oder "krank", z.B. mithilfe der Koronarangiografie) vorliegt, das KI-System bzw. das neuronale Netz mit "frischen" Gesundheitsparametern speist. Neue Messdaten helfen die Gewichtungen des Neuronalen Netzes weiter zu optimieren, sodass mit zunehmenden Daten eine immer bessere Klassifizierung zwischen krank und gesund stattfinden kann. Die charakteristischen Gewichtungen zwischen den Neuronen im KI-System werden mit jedem neuen Patienten aus dem Trainingsset angepasst, sodass Fehler vergangener Diagnosen minimiert werden.

Somit lernt das KI-System bzw. das neuronale Netz mit jeder Information dazu und optimiert sich dadurch permanent selbst, falls vor der eigentlichen Diagnose eines Patienten zuvor ein Training des KI-Systems durchgeführt wird, nämlich auf Grundlage der Daten von Personen, von denen der Gesundheitszustand eindeutig bekannt ist. Im Ergebnis wird hierbei die Diagnose eines einzelnen mit den Diagnosen von unzähligen Sensorinformationen/Anwendern (auch historisch) abgeglichen. Die Genauigkeit und damit die Aussagefähigkeit der Messergebnisse werden somit - im Trainingsmodus - permanent optimiert und verbessern sich mit jeder einzelnen Messung, nämlich in Bezug auf eine Person, deren Gesundheitszustand bekannt ist. Diesbezüglich wird gesondert darauf hingewiesen, dass es sich bei dem KI-System bzw. einem neuronalen Netz nicht um ein einzelnes System bzw. Netz handelt, sondern um eine stetig steigende Zahl von solchen Systemen bzw. Netzen. Entsprechend sieht die Erfindung die Einbindung einer Mehrzahl von solchen KI-Systemen bzw. neuronalen Netzen vor, zum Beispiel in Form eines umfassenden großen neuronalen Netzes, welches auf einer ständig wachsenden Anzahl von neuronalen Netzen basiert.

Der vorstehend genannte Aspekt des Trainings mittels spezifischer Lernwerte gilt auch für die erfindungsgemäße Einrichtung. Dies bedeutet, dass deren KI-System bzw. neuronales Netz in der erläuterten Weise durch Eingabe von spezifischen Lernwerten trainiert ist, bevor dann in der Praxis die Untersuchung eines Patienten und eine anschließende Diagnose auf Grundlage der ermittelten Messwerte erfolgt.

Das Trainieren des KI-Systems bzw. eines neuronalen Netzes durch Eingabe der besagten spezifischen Lernwerte erfolgt auf der Grundlage, dass hierbei EKG-Signale an Patienten aufgenommen bzw. verarbeitet werden, zu denen eine eindeutige Kenntnis/ Information bezüglich des Gesundheitszustands ("gesund" oder "krank", z.B. mithilfe der Koronarangiografie) vorliegt. Weitere Details bezüglich der Durchführung eines solchen Trainings werden nachfolgend im Zusammenhang mit einer zugehörigen Ausführungsform der Erfindung noch gesondert erläutert.

Die vorliegende Erfindung sieht auch ein Verfahren zur Ermittlung und Darstellung eines auf das Herz bezogenen Raumvektors vor, der den Vektor des elektrischen Feldes darstellt, das durch die Aktivität des Herzens gebildet wird.

Hierbei werden Messwerte des Herzens am Körper an einem ersten Ableitungspunkt, an einem zweiten Ableitungspunkt, an einem dritten Ableitungspunkt und an einem vierten Ableitungspunkt erfasst, wobei Potentialdifferenzen in Form einer anterioren Ableitung zwischen dem ersten Ableitungspunkt und dem vierten Ableitungspunkt, einer dorsalen Ableitung zwischen dem zweiten Ableitungspunkt und dem vierten Ableitungspunkt, einer horizontalen Ableitung zwischen dem dritten Ableitungspunkt und dem vierten Ableitungspunkt, einer vertikalen Ableitung zwischen dem ersten Ableitungspunkt und dem dritten Ableitungspunkt, und einer inferioren Ableitung zwischen dem ersten Ableitungspunkt und dem zweiten Ableitungspunkt gemessen werden. Es werden ein orthogonales System mit den Beziehungen:

$$x = D \cos 45° - I$$

$$y = D \sin 45° + A$$

$$z = (V - H) \sin 45°$$

gebildet und die Messwerte und der daraus ermittelte Raumvektor in diesem orthogonalen System (x, y, z) abgebildet. Im Anschluss daran werden bei diesem Verfahren folgende Schritte durchlaufen:

(a) Durchführung einer Messung an einem Patienten unter Verwendung der ersten bis vierten Ableitungspunkte am Körper des Patienten, um damit für diesen Patienten ein Kardiogramm zu gewinnen,

(b) Extrahieren der Amplituden der R-Welle aus dem Kardiogramm von Schritt (a) für jeden Herzschlag in x- , y- und z-Richtung,

(c) Ermitteln von Mittelwerten $\mu_x$, $\mu_y$, $\mu_z$ und Standard-Abweichungen $\sigma_x$, $\sigma_y$, $\sigma_z$ der jeweils in Millivolt erfassten Amplituden aus dem Kardiogramm gemäß Schritt (b), wobei mit diesen Mittelwerten $\mu_x$, $\mu_y$, $\mu_z$ und Standard-Abweichungen $\sigma_x$, $\sigma_y$, $\sigma_z$ dann ein Berechnungsvektor gebildet wird,

(d) Bilden einer Koeffizientenmatrix, die auf Grundlage einer Hauptachsentransformation für Messungen an Vergleichs-Patienten mit unterschiedlichen Anlageschemata gewonnen worden ist,

(e) Multiplizieren des Berechnungsvektors von Schritt (c) mit der Koeffizientenmatrix von Schritt (d), zur Bildung eines Ergebnisvektors mit insgesamt sechs Hauptachsen,

(f) Extrahieren der ersten Hauptachse und der zweiten Hauptachse aus dem Ergebnisvektor von Schritt (e), zur Bildung eines Referenzpunktes im Raum der ersten und zweiten Hauptachse,

(g) Ermitteln eines euklidischen Abstands des Referenzpunktes von einem vorbestimmten Zielpunkt, der einer korrekten Position der vier Ableitungspunkte am menschlichen Körper entspricht,

(h) falls der Abstand des Referenzpunktes von dem vorbestimmten Zielpunkt größer als ein vorbestimmter Maximalwert ist: Durchführen einer Winkelkorrektur für ein aus dem ersten Ableitungspunkt, dem dritten Ableitungspunkt und dem vierten Ableitungspunkt gebildetes erstes Dreieck und für ein aus dem ersten Ableitungspunkt, dem zweiten Ableitungspunkt und dem vierten Ableitungspunkt gebildetes zweites Dreieck, so dass damit der euklidische Abstand zwischen dem Referenzpunkt und dem vorbestimmten Zielpunkt durch Anpassung des orthogonalen Systems (x, y, z) an die geänderte Geometrie minimiert wird.

Das zuletzt genannte Verfahren nach der Erfindung beruht auf der wesentlichen Erkenntnis, dass es auf Grundlage einerseits der Kenntnis einer korrekten Position für die vier Ableitungspunkte und andererseits einer Hauptachsentransformation möglich ist, einen nicht korrekten Sitz einer Elektrode, die insbesondere dem ersten Ableitungspunkt zugewiesen ist, zu kompensieren, so dass die gewonnenen Messwerte des Patienten weiterhin verwendet werden können und eine realistische Diagnose für den untersuchten Patienten ermöglichen.

In vorteilhafter Weiterbildung der Erfindung kann bei dem zuletzt genannten Verfahren vorgesehen sein, dass vor Durchführung des Schritts (h) der vorbestimmte Zielpunkt aus einem Zielgebiet ausgewählt wird, das auf Grundlage von Mittelwerten und Standardabweichungen einer Mehrzahl von Messungen gebildet wird. Dies bedeutet, dass mit diesen Messungen Vergleichswerte für die Positionen der Elektroden am menschlichen Körper gewonnen werden, um damit die Winkelkorrektur gemäß Schritt (h) durchzuführen.

Zur Überwachung des Herzens insbesondere bei einem Menschen ist gemäß der vorliegenden Erfindung auch ein Verfahren vorgesehen, bei dem EKG-Signale am Herzen aufgenommen und auf Grundlage dessen ein Raumvektor anhand der Vektorkardiographie bestimmt wird, wobei dieser Raumvektor den zeitlichen Verlauf des Summenvektors des elektrischen Feldes des Herzens abbildet und eine der Feldrichtung entsprechende Richtung und eine dem Potential entsprechende Länge aufweist. Es wird ein Quotient aus den Flächen, die von einer Länge des Raumvektors (= Radiusvektor) als Funktion der Zeit jeweils während der R-Welle und während der T-Welle überstrichen werden, gebildet, wobei anschließend dieser dimensionslose und skalare Quotient einer weiteren Auswertung zugeführt wird. Im Zuge dieser weiteren Auswertung wird der Quotient gebildet aus der Fläche (R-Welle)/Fläche (T-Welle) jeweils in Bezug zu vorbestimmten Grenzwerten gesetzt, die eine Relevanz für das zu untersuchende Krankheitsbild aufweisen. Dies bedeutet, dass der besagte Quotient gebildet aus der Fläche (R-Welle)/Fläche (T-Welle) mit zumindest einem vorbestimmten Grenzwert korreliert bzw. verglichen wird, auf Grundlage dessen dann eine Diagnose für den untersuchten Patienten in Hinblick auf ein bestimmtes Krankheitsbild (z.B. KHK und/oder HRS und/oder Herzklappeninsuffizienz eines Patienten ) erstellt wird bzw. möglich ist.

In vorteilhafter Weiterbildung des zuletzt genannten Verfahrens wird für das untersuchte Herz eine koronare Herzkrankheit (KHK) erkannt bzw. festgestellt, falls der aus den von dem Raumvektor jeweils während der R-Welle und während der T-Welle überstrichenen Flächen gebildete Quotient

$$\frac{\text{Fläche (R-Welle)}}{\text{Fläche (T-Welle)}} = a$$

nicht im Intervall $[a_{0,KHK}, a_{1,KHK}]$ bzw. ausserhalb dieses Intervalls liegt. Dieses Intervall wird durch einen unteren Grenzwert $a_{0,KHK}$ und durch einen oberen Grenzwert $a_{1,KHK}$ definiert. Diese Grenzwerte $a_{0,KHK}$ und $a_{1,KHK}$ können in Abhängigkeit von einem Trainingsset bestimmt werden. Des Weiteren wird darauf hingewiesen, dass die Indizierung "KHK" des unteren und oberen Grenzwerts $a_{0,KHK}$ und $a_{1,KHK}$ für die vorliegende Erfindung dahingehend zu verstehen ist, dass mit Hilfe dieser Grenzwerte das Krankheitsbild einer koronaren Herzkrankheit ("KHK") erkannt werden kann, die auch unter der Bezeichnung ischämische Herzkrankheit ("IHK") bekannt ist.

Bei dem soeben diskutierten Verfahren nach der vorliegenden Erfindung kann für das untersuchte Herz auch eine Herzrhythmusstörung (HRS) erkannt werden, falls der aus den von dem Raumvektor (10) jeweils während der R-Welle und während der T-Welle überstrichenen Flächen gebildete Quotient die Bedingung

$$\frac{\text{Fläche (R-Welle)}}{\text{Fläche (T-Welle)}} < a_{0,\,HRS}$$

oder

$$\frac{\text{Fläche (R-Welle)}}{\text{Fläche (T-Welle)}} > a_{1,HRS}$$

erfüllt.

Entsprechend ist es nach dem zuletzt diskutierten Verfahren gemäß der vorliegenden Erfindung möglich, durch einen Vergleich des Quotienten gebildet aus den Flächen, die von einer Länge des Raumvektors (= Radiusvektor) als Funktion der Zeit jeweils während der R-Welle und während der T-Welle überstrichen werden, mit vorbestimmten Werten für den Parameter "a" eine zutreffende Erstdiagnose für das untersuchte Herz eines Patienten zu erstellen, im Hinblick auf ein mögliches Vorliegen einer HRS.

In Abhängigkeit davon, ob konkret das Vorliegen einer HRS oder oder KHK festgestellt werden soll, versteht sich, dass die Grenzwerte $a_0$ und $a_1$ zum Erkennen dieser Krankheitsbilder und damit für einen Vergleich bzw. eine Korrelation mit dem Quotienten gebildet aus der Fläche (R-Welle)/Fläche (T-Welle), der auf entsprechenden Daten basiert, geeignet sind. Im Zusammenhang mit einer HRS kann dies durch die Indizierung "HRS" ausgedrückt werden (d.h. durch $a_{0,HRS}$ , $a_{1,HRS}$ ); im Zusammenhang mit einer KHK kann dies durch die Indizierung "KHK" ausgedrückt werden (d.h. durch $a_{0,KHK}$ , $a_{1,KHK}$ ).

Falls in Bezug auf ein zu untersuchendes Krankheitsbild (z.B. HRS und/oder KHK) belastbare Daten für die unteren und oberen Grenzwerte $a_0$, $a_1$ vorliegen, können diese Grenzwerte vor der Untersuchung eines Patienten auf diese vorbestimmten Werte festgelegt werden, jeweils in Abhängigkeit davon, ob das Vorliegen einer HRS oder einer KHK untersucht werden soll. Alternativ hierzu ist es möglich, diese Grenzwerte mit Hilfe von einem Trainingsset von Datensätzen einer Vielzahl von Probanden zu verwenden, wie nachstehend im Detail erläutert.

In vorteilhafter Weiterbildung der Erfindung können die unteren und oberen Grenzwerte $a_0$ und $a_1$ jeweils mit Hilfe eines Trainingssets von Probanden-Daten bestimmt werden. Hierbei werden folgende Schritte durchgeführt:

(i) Berechnung des Quotienten Fläche (R-Welle) / Fläche (T-Welle) für alle Probanden, um damit Ratio-Zeitreihen zu erhalten,
(ii) Berechnung eines Mittelwertes ($\mu$) und einer zugehörigen Standardabweichung (o) aus den Ratio-Zeitreihen von Schritt (i),
(iii) Bestimmung des unteren Grenzwertes $a_0$ unter Berücksichtigung des Mittelwertes ($\mu$) und der Standardabwei-

chung (σ) von Schritt (ii), durch die Beziehung:

$$a_0 = \text{Mittelwert } (\mu) - \text{Standardabweichung } (\sigma),$$

und/oder
Bestimmung des oberen Grenzwertes $a_1$ unter Berücksichtigung des Mittelwertes ($\mu$) und der Standardabweichung ($\sigma$) von Schritt (ii), durch die Beziehung:

$$a_1 = \text{Mittelwert } (\mu) + \text{Standardabweichung } (\sigma).$$

In Bezug auf die vorstehend genannten Schritte (i) bis (iii) versteht sich, dass diese einem Training zuzuordnen sind, mit dem die unteren und oberen Grenzwerte $a_0$ und $a_1$ mit größerer "Belastbarkeit" bestimmt werden können, um anschließend und auf Grundlage dessen eine tatsächliche Diagnose eines Patienten für das untersuchte Krankheitsbild mit größerer Genauigkeit zu erstellen.

Zur Klarstellung der vorstehend genannten Rechenvorschrift in Schritt (iii) sei nochmals darauf hingewiesen, dass der untere Grenzwert $a_0$ dadurch gebildet wird, dass der Mittelwert $\mu$ und die zugehörige Standardabweichung $\sigma$ voneinander subtrahiert werden. Demgegenüber wird der obere Grenzwert $a_1$ dadurch gebildet, dass der Mittelwert $\square$ und die zugehörige Standardabweichung $\sigma$ miteinander addiert werden.

In Bezug auf die vorstehend genannte Bestimmung der unteren und oberen Grenzwerte $a_0$ und $a_1$ versteht sich, dass die zugrunde liegenden Daten der Probanden, mit denen gemäß Schritt (i) die Ratio-Zeitreihen gebildet werden, sich jeweils auf das zu untersuchende Krankheitsbild HRS und/oder KHK beziehen bzw. hierfür relevant sind. Beispielsweise werden zur Untersuchung des Vorliegens einer koronaren Herzkrankheit ("KHK") die Grenzwerte $a_{0,KHK}$ und $a_{1,KHK}$ lediglich anhand solcher Datensätze von Probanden bestimmt, von denen mit sicherer Kenntnis ausgeschlossen werden kann, dass sie an einer KHK bzw. IHK erkrankt sind. Mutatis mutandis gilt dies auch für die Grenzwerte $a_{0,HRS}$, $a_{1,HRS}$ bei der Untersuchung eines Patienten hinsichtlich des Vorliegens einer HRS.

Gemäß einer vorteilhaften Weiterbildung der Erfindung ist vorgesehen, dass zur Bestimmung des unteren und oberen Grenzwerts lediglich die Datensätze von gesunden Patienten ("- -") verwendet werden. Im Vergleich zu den Datensätzen von kranken Patienten hat dies den Vorteil, dass die Standardabweichungen der Mittelwerte von den gesunden Patienten in der Regel geringer sind, wodurch das Erkennen der Krankheitsbilder bzw. Befunde HRS und/oder KHK mit größerer Genauigkeit bzw. Zuverlässigkeit möglich ist.

In vorteilhafter Weiterbildung der Erfindung, insbesondere in Bezug auf alle der vorstehend genannten Verfahren, kann vorgesehen sein, dass zur Aufnahme der EKG-Signale ein T-Shirt verwendet wird, welches vier Sensoren bzw. Elektroden aufweist, die einer korrekten Position der vier Ableitungspunkte am Körper des Patienten zugeordnet sind. Diese Elektroden können in den Stoff des T-Shirts geeignet eingearbeitet sein. In gleicher Weise wie ein T-Shirt kann für die Sensoren bzw. Elektroden, die in Kontakt mit dem Körper des Patienten zu bringen sind, auch eine tragbare bzw. mobile Sensorik vorgesehen sein, z.B. in Form eines Brustgurtes oder dergleichen. Wichtig hierbei ist, dass bei einer solchen tragbaren Sensorik die einzelnen Sensoren bzw. Elektroden zu einem Verbund miteinander verbunden sind und hierdurch auch eine genaue Positionierung an den jeweiligen Ableitungspunkten am Körper des Patienten erreicht wird. Entsprechend erübrigt sich damit ein manuelles Anlegen der einzelnen Sensoren am Körper des Patienten.

Bei dem bzw. den vorstehend genannten Verfahren gemäß der vorliegenden Erfindung handelt es sich um einen nicht-invasiven, reproduzierbaren, schnell durchführbaren und kostengünstigen Diagnose-Ansatz zur Erkennung hämodynamisch relevanter Stenosen der Koronararterien in Ruhe. Die Diagnose erfolgt über eine computerbasierte infinitesimale, dreidimensionale Verrechnung der Erregungsabläufe des Säugerherzens basierend auf einem spezifischen Algorithmus in Verbindung mit einem KI-System bzw. einem neuronalen Netzwerk in Korrelation zur intrinsischen Blutversorgung sowie der spezifischen räumlichen Ausrichtung des Myokards im Dipolfeld als Funktion der Zeit ausgehend von einem definierten Punkt. Mit einem erfindungsgemäßen Verfahren ist es möglich, in Bezug auf die untersuchten Patienten eine Sensitivität von 95 bis über 99% und eine Spezifität von 80 bis über 90% zu erreichen. Dieser Ansatz wird von der Anmelderin auch als "Cardisiographie" bezeichnet.

In Bezug auf die vorliegende Erfindung wird gesondert hervorgehoben, dass deren Durchführung vorzugsweise unter Verwendung eines Computers oder vergleichbarer Rechnereinheiten erfolgt. Dies bedeutet, dass beispielsweise das System basierend auf künstlicher Intelligenz (KI), welches bei einem erfindungsgemäßen Verfahren von Anspruch 1 und der entsprechenden Einrichtung von Anspruch 14 zur Früherkennung des Vorliegens einer KHK und/oder einer HRS verwendet wird, mit Hilfe eines Computers oder dergleichen zum Einsatz kommt. Mutatis mutandis gilt dies auch für ein Verfahren gemäß Anspruch 20 insbesondere in Bezug auf dessen Schritte (b) bis (h), und auch für ein Verfahren gemäß Anspruch 28 in Bezug auf die Auswertung des Quotienten gebildet aus der Fläche (R-Welle)/Fläche (T-Welle) vorzugsweise unter Berücksichtigung von zumindest einem vorbestimmten Grenzwert.

Nachstehend sind verschiedene Ausführungsformen der Erfindung anhand einer schematisch vereinfachten Zeichnung im Detail beschrieben.

Es zeigen:

| | | |
|---|---|---|
| Fig. 1 | | eine vereinfachte Prinzipskizze einer erfindungsgemäßen Einrichtung zur Früherkennung des Vorliegens einer KHK oder von HRS, |
| Fig. 2 | | die Ansicht eines Patienten von vorne (Fig. 2a) und von hinten (Fig. 2b), |
| Fig. 3 | | die Ansicht eines T-Shirts von vorne (Fig. 3a) und von hinten (Fig. 3b), das bei der Einrichtung gemäß Fig. 1 verwendbar ist, |
| Fig. 4, Fig. 7b | | jeweils die Ansicht eines Patienten von vorne, zur Verdeutlichung eines orthogonalen Systems (x, y, z), in dem gemäß der vorliegenden Erfindung ein Raumvektor abgebildet wird, |
| Fig. 5 | | schematische vereinfachte Ansichten eines ersten Dreiecks (Fig. 5a) und eines zweiten Dreiecks (Fig. 5b), auf Grundlage derer in Bezug auf das orthogonale System nach Fig. 4 erfindungsgemäß eine Winkelkorrektur durchgeführt wird, |
| Fig. 6 | | eine prinzipiell vereinfachte Ansicht einer erfindungsgemäßen System-Architektur, innerhalb derer die erfindungsgemäße Einrichtung von Fig. 1 eingesetzt wird, |
| Fig. 7a | | eine prinzipiell vereinfachte Darstellung eines Raumvektors, der bei einer Aktivität des menschlichen Herzens gebildet wird, |
| Fig. 8 | | ein Ablaufdiagramm, mit dem ein Verfahren nach der vorliegenden Erfindung durchgeführt wird, |
| Fig. 9 | | ein Ablaufdiagramm, mit dem ein Verfahren nach der vorliegenden Erfindung gemäß einer weiteren Ausführungsform durchgeführt wird, |
| Fig. 10 | | eine prinzipiell vereinfachte Darstellung einer 3D-Matrix (Fig. 10a) von Messwerten bzw. eine normierte Version hiervon (Fig. 10b), mit den aus den Parameter-Zeitreihen bzw. Parametern abgeleiteten Statistiken, die bei einem Verfahren gemäß Fig. 8 bzw. Fig. 9 zur Anwendung kommt, |
| Fig. 11a-11d | | beispielhafte Zeitreihen-Parameter, die bei einem Verfahren gemäß Fig. 9 anwendbar sind, |
| Fig. 12 | | beispielhafte statistische Verfahren, die bei einem Verfahren gemäß Fig. 9 anwendbar sind, |
| Fig. 13 | | ein Ablaufdiagramm, mit dem ein Verfahren nach der vorliegenden Erfindung durchgeführt wird, |
| Fig. 14 | | ein weiteres Ablaufdiagramm, mit dem ein Verfahren nach der vorliegenden Erfindung gemäß einer weiteren Ausführungsform durchgeführt wird, |
| Fig. 15 | | die Multiplikation einer Berechnungsmatrix mit einem Berechnungsvektor nach einem weiteren Verfahren der vorliegenden Erfindung, |
| Fig. 16 | | eine prinzipiell vereinfachte Hauptachsentransformation für das Verfahren von Fig. 15, |
| Fig. 17 | | eine vereinfachte Veranschaulichung der Bildung eines Quotienten aus den Flächen, die von einer Länge des Raumvektors (= Radiusvektor) als Funktion der Zeit jeweils während der R-Welle und während der T-Welle überstrichen werden, und |
| Fig. 18 | | eine vereinfachte Veranschaulichung einer Mehrzahl von neuronalen Netzen, die bei einer Anwendung bzw. Durchführung der vorliegenden Erfindung zum Einsatz kommen. |

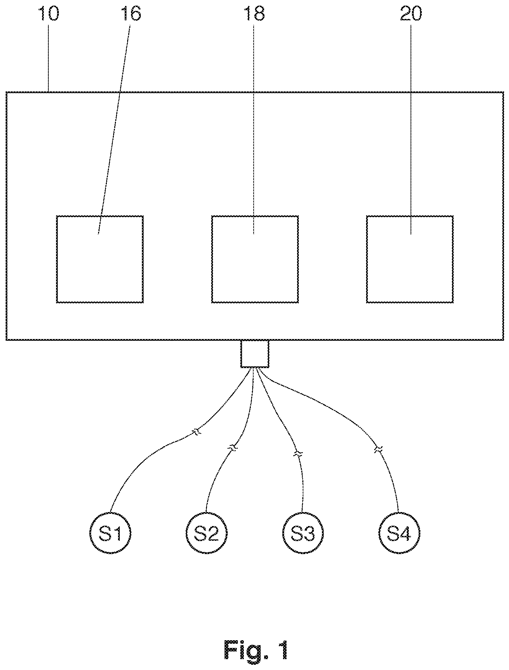

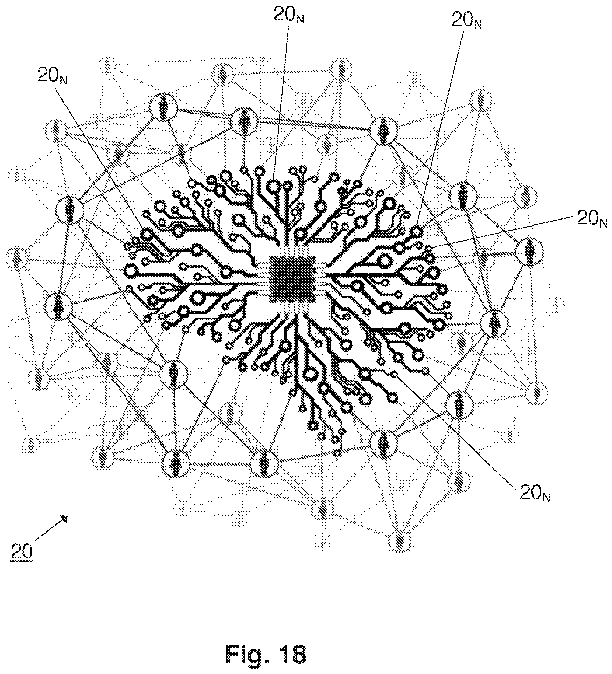

Nachfolgend sind unter Bezugnahme auf die Fig. 1-18 bevorzugte Ausführungsformen von erfindungsgemäßen Herzüberwachungsverfahren und einer hierzu eingesetzten Einrichtung 10 im Detail dargestellt und erläutert. Gleiche Merkmale in der Zeichnung sind jeweils mit gleichen Bezugszeichen versehen. An dieser Stelle wird gesondert darauf hingewiesen, dass Darstellungen in der Zeichnung lediglich vereinfacht und insbesondere ohne Maßstab gezeigt sind.

Die Einrichtung 10, in Fig. 1 prinzipiell vereinfacht dargestellt, dient bei der Untersuchung eines Patienten 11 (vgl. Fig. 2) zur Früherkennung des Vorliegens einer Koronaren Herzkrankheit (KHK) oder einer Herzrhythmusstörung (HRS). Hierzu umfasst die Einrichtung 10 vier Sensoren bzw. Elektroden S1, S2, S3, S4, einen datentechnischen Filter 16, eine Auswerteeinrichtung 18 und ein System 20 basierend auf Künstlicher Intelligenz (KI). Die Auswerteeinrichtung 18 ist mit einem (nicht gezeigten) Speicherelement ausgestattet, so dass darin Messsignale bzw. -werte zumindest kurzzeitig gespeichert werden können. Das KI-System 20 kann zumindest ein neuronales Netz $20_N$ (vgl. auch Fig. 18) oder eine Mehrzahl solcher neuronalen Netze aufweisen bzw. aus solchen neuronalen Netzen gebildet sein.

Die Sensoren S1-S4 sind signaltechnisch (z.B. über eine Kabelverbindung, oder über eine drahtlose Funkstrecke) derart an die Einrichtung 10 angeschlossen, dass deren Messwerte zunächst den Filter 16 durchlaufen und anschließend in die Auswerteeinrichtung 18 gelangen. Die Auswerteeinrichtung 18 ist datentechnisch mit dem KI-System 20 verbunden, derart, dass die Messgrößen, die mittels der Auswerteeinrichtung 18 geeignet aufbereitet bzw. in orthogonalisierte Messgrößen auf Grundlage der Vektorkardiographie überführt werden, wie nachstehend noch gesondert ist, in das KI-System 20 eingegeben werden können. Dies erfolgt zum Zwecke einer Diagnose für den Patienten 11, der unter Verwendung der Einrichtung 10 untersucht wird.

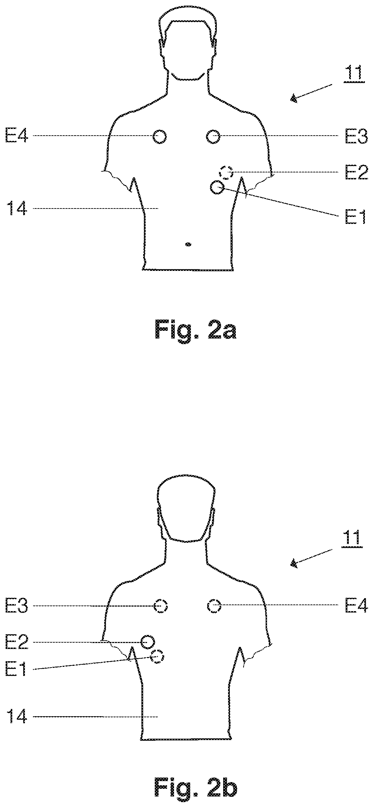

Fig. 2 zeigt - zum verbesserten Verständnis der Erfindung - einen Patienten 11, nämlich in einer Ansicht von vorne (Fig. 2a) und in einer Ansicht von hinten (Fig. 2b). Am Körper 14 des Patienten 11 sind insgesamt vier Ableitungspunkte vorgesehen, nämlich ein erster Ableitungspunkt E1, ein zweiter Ableitungspunkt E2, ein dritter Ableitungspunkt E3 und ein vierter Ableitungspunkt E4. Die Ableitungspunkte E1, E3 und E4 befinden sich jeweils im Brustbereich des Patienten 11, wobei der Ableitungspunkt E2 sich im Rückenbereich des Patienten 11 befindet. In Bezug auf diese vier Ableitungspunkte E1 bis E4 ist zu beachten, dass der erste Sensor S1 an dem ersten Ableitungspunkt E1, der zweite Sensor S2 an dem zweiten Ableitungspunkt E2, der dritte Sensor S3 an dem dritten Ableitungspunkt E3 und der vierte Sensor S4 an dem vierten Ableitungspunkt E4 am Körper 14 des Patienten 11 angebracht werden. Zwischen diesen Ableitungspunkten werden jeweils Potentialdifferenzen gemessen, wie nachstehend noch gesondert erläutert. Bezüglich weiterer Details zu den Positionen dieser einzelnen Ableitungspunkte E1-E4 am Körper 14 des Patienten wird auf die Offenbarung gemäß EP 86 429 B1, auf deren Inhalt hiermit in vollem Umfang Bezug genommen wird.

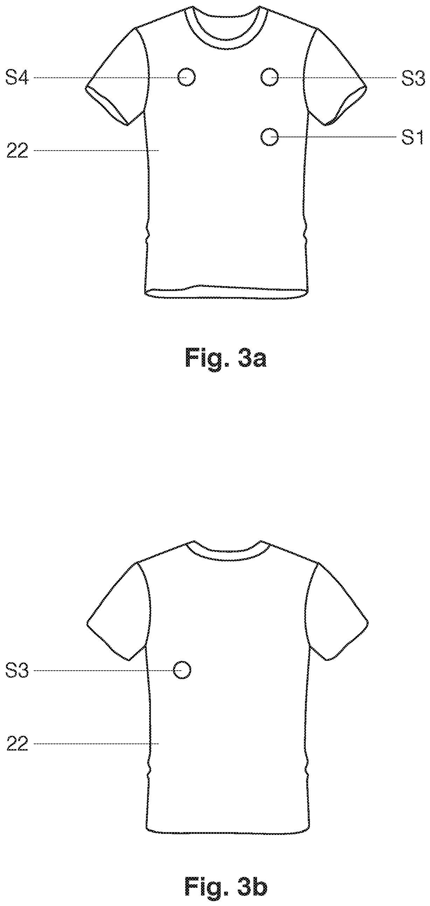

In der Fig. 3 ist vereinfacht ein T-Shirt 22 gezeigt. Die vorstehend genannten Sensoren S1-S4 der Einrichtung 10 können in das T-Shirt 22 integriert sein, zum Beispiel durch ein Verweben in dessen textiler Struktur. Ein solches T-Shirt 22 führt zu dem Vorteil, dass ein Patient 11 zur Vorbereitung einer Untersuchung lediglich dieses T-Shirt 22 anzieht bzw. überstreift, wobei dann die in das T-Shirt 22 integrierten Sensoren S1-S4 automatisch in ihre bestimmungsgemäße Position angrenzend zu den vier Ableitungspunkten E1-E4 gelangen. Durch den Einsatz eines solchen T-Shirts 22 erübrigt sich ein zeitaufwendiges und ggf. fehlerträchtiges manuelles Anlegen der einzelnen Sensoren S1-S4 am Körper 14 des Patienten 11. Alternativ zu dem T-Shirt 22 kann auch ein (nicht gezeigter) Brustgurt verwendet werden, an dem die Sensoren bzw. Elektroden S1-S4 angebracht sind.

Zur Untersuchung eines Patienten 11 bzw. zur Gewinnung eines Probedatensatzes zum Training des KI-Systems 20 werden die vier Sensoren S1-S4 an den zugeordneten vier Ableitungspunkten E1-E4 am menschlichen Körper 14 positioniert. Im Anschluss daran werden im Ruhezustand des Patienten 11 mit Hilfe der in Position gebrachten Sensoren S1-S4 EKG-Signale am Herzen 12 des Patienten 11 aufgenommen. Die EKG-Signale werden sodann durch den Filter 16 geeignet gefiltert, und anschließend in der Auswerteeinrichtung 18 in orthogonalisierte Messgrößen (in die Achsen x, y, z) nach dem Sanz'schen System gemäß EP 86 429 B1 transformiert.

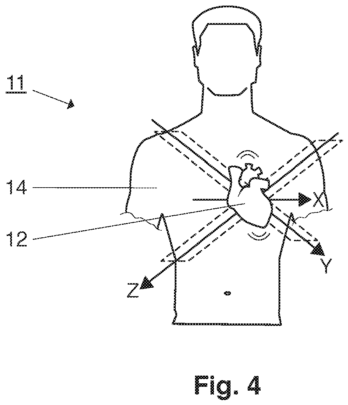

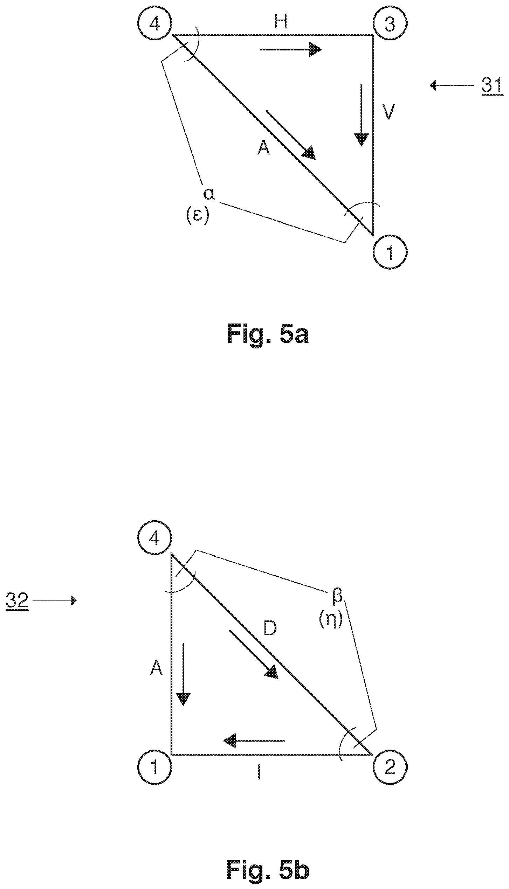

In der Fig. 4 sind die Achsen x, y, z nach dem Sanz'schen System veranschaulicht, in Bezug auf den Körper 14 eines Patienten 11 und dessen Herz 12. Diesbezüglich wird auch auf die Fig. 5 verwiesen, wobei in Fig. 5a schematisch ein erstes Dreieck 31 veranschaulicht ist, welches zwischen den Ableitungspunkten E1, E3 und E4 gebildet wird, und in Fig. 5b ein zweites Dreieck 32 veranschaulicht ist, welches zwischen den Ableitungspunkten E1, E2 und E4 gewählt ist. Die Bedeutung dieser beiden Dreiecke 31, 32 wird nachfolgend noch gesondert erläutert.

Wie bereits erläutert, werden zwischen den einzelnen Ableitungspunkten E1-E4 jeweils Potentialdifferenzen erfasst. Im Einzelnen sind dies eine anteriore Ableitung A zwischen dem ersten Ableitungspunkt E1 und dem vierten Ableitungspunkt E4, eine dorsale Ableitung D zwischen dem zweiten Ableitungspunkt E2 und dem vierten Ableitungspunkt E4, eine horizontale Ableitung H zwischen dem dritten Ableitungspunkt E3 und dem vierten Ableitungspunkt E4, eine vertikale Ableitung V zwischen dem ersten Ableitungspunkt E1 und dem dritten Ableitungspunkt E3, und schließlich eine inferiore Ableitung I zwischen dem ersten Ableitungspunkt E1 und dem zweiten Ableitungspunkt E2. Aus dem ersten Ableitungspunkt E1, dem dritten Ableitungspunkt E3 und dem vierten Ableitungspunkt E4 wird - ausweislich der Darstellung in Fig. 5a - ein erstes Dreieck 31 gebildet, wobei aus dem ersten Ableitungspunkt E1, dem zweiten Ableitungspunkt E2 und dem vierten Ableitungspunkt E4 - ausweislich der Darstellung in Fig. 5b - ein zweites Dreieck 32 gebildet wird. Die genannten Ableitungen sind mit ihren Bezeichnungen A, D, H, V und I ebenfalls in den Darstellungen von Fig. 5a und Fig. 5b gezeigt. Für weitere Zusammenhänge in Bezug auf diese Ableitungen darf an dieser Stelle auf den Inhalt der EP 86 429 B1 verwiesen werden.

Die Bedeutung der ersten und zweiten Dreiecke 31, 32 ist nachfolgend noch an anderer Stelle im Zusammenhang mit einem sog. "Korrektur-Verfahren" gemäß der vorliegenden Erfindung gesondert erläutert.

Bei Durchführung einer EKG-Messung gelangen die elektrischen Messwerte der vorstehend genannten Ableitungen A, D, H, V und I in die Einrichtung 10 und werden darin, wie oben bereits erläutert, entsprechend weiter verarbeitet.

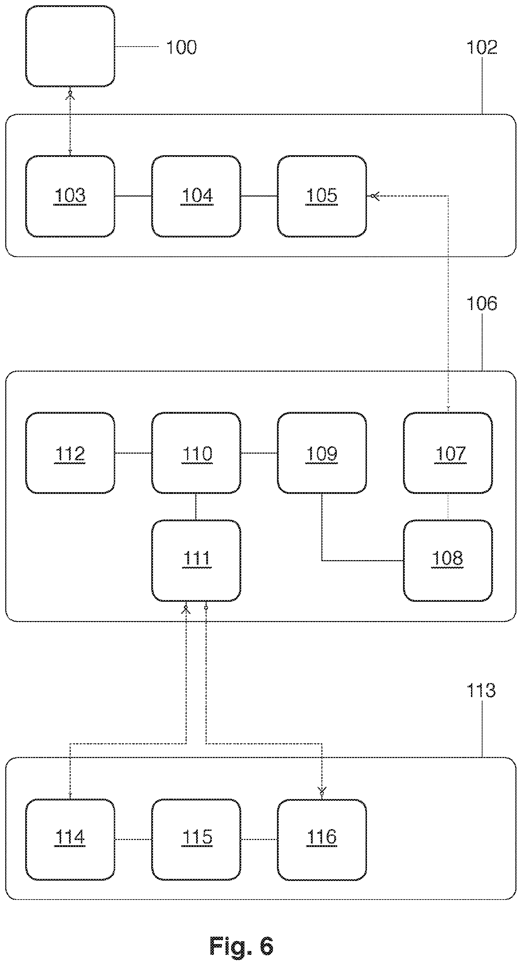

Unter Bezugnahme auf die Fig. 6 werden nachfolgend weitere Einzelheiten der Einrichtung 10 und deren Einbindung in eine erfindungsgemäße Gesamtarchitektur zur Durchführung der vorliegenden Erfindung erläutert. Hierzu im Einzelnen:

Die Architektur gemäß Fig. 6 sieht folgende Komponenten vor: Sensoreinrichtung 100, Datenaufzeichner 102, Cardisio®-Vorrichtung 106 und Server 113. Der Datenaufzeichner 102 umfasst einen Signalempfänger 103, einen Signalkonverter 104 und einen Signalspeicher 105. Die Cardisio-Vorrichtung 106 umfasst einen Signalleser 107, einen Vektordaten-Generator 108, einen Vektordaten-Speicher 109, einen Vektordaten-Auswerter 110, einen Vektordaten-Synchronisierer 111 und eine Vektordaten-Anzeige 112. Der Server umfasst 113 einen Vektordaten-Speicher 114, einen Vektordaten-Analysator 115 und einen Vektordaten-Auswerte-Speicher 116.

Die Sensoreinrichtung 100 wird durch die vorstehend genannten vier Sensoren bzw. Elektroden S1-S4 der Einrichtung 10 ausgebildet.

Die Sensoreinrichtung 100 und der Datenaufzeichner 102 bilden die Komponenten bzw. Bauteile der Einrichtung 10, die zur Messung bzw. Aufnahme der EKG-Signale dienen. Hiermit werden die analogen EKG-Signale empfangen, verarbeitet und geeignet in digitale Signale transformiert. Wie bereits erläutert, können die digitalen Signale zumindest kurzzeitig in dem Speicherelement der Auswerteeinrichtung 18 - vorliegend in Form des Signalspeichers 105 - gespeichert werden.

Die Cardisio-Vorrichtung 106 liest die digitalen Signale von dem Datenaufzeichner 102, zwecks einer Bestimmung von Vektordaten auf Grundlage der digitalen Signale mittels des Vektordaten-Generators 108. Die hierbei erzeugten Vektordaten werden anschließend in dem Vektordaten-Speicher 109 gespeichert. Ausgehend hiervon erzeugt der Vektordaten-Auswerter 110 eine Darstellung dieser Vektordaten z.B. in Form einer dreidimensionalen Kurve, wobei diese Darstellung dann mittels der Vektordaten-Anzeige 112 gezeigt bzw. anschaulich gemacht wird.

Die Architektur gemäß Fig. 6 veranschaulicht des Weiteren, dass innerhalb der Cardisio-Vorrichtung 106 der Vektordaten-Auswerter 110 signaltechnisch mit dem Vektordaten-Synchronisierer 111 verbunden ist, und Letzterer über eine Signalstrecke bzw. -verbindung an den Server 113 angeschlossen ist. Hierdurch können die erzeugten Vektordaten über den Vektordaten-Synchronisierer 111 zunächst in den Vektordaten-Speicher 114 auf dem Server 113 eingelesen werden. Im Anschluss daran ist es mittels des Vektordaten-Analysators 115 möglich, eine gezielte Auswertung von größeren Datenmengen bzw. der erzeugten Vektordaten vorzunehmen und diesbezüglich statistische Analysen durchzuführen. Schlussendlich werden die manuellen und/oder automatischen Auswertungen der Datensätze in dem Vektordaten-Auswerte-Speicher 116 gespeichert, wobei ausgehend hiervon diese Auswertungen wieder auf die Vorrichtung 106 hochgeladen werden können, z.B. zwecks einer Darstellung auf bzw. mit der Vektordaten-Anzeige 112.

Für die anhand der Fig. 6 erläuterten Komponenten und Bauteile der Sensoreinrichtung 100, des Datenaufzeichners 102, der Cardisio-Vorrichtung 106 und des Servers 113 versteht sich, dass diese jeweils Teile der erfindungsgemäßen Einrichtung 10 (vgl. Fig. 1) bilden. Insbesondere der Vektordaten-Auswerte-Speicher 116 ist Teil des KI-Systems 20 bzw. eines neuronalen Netzes $20_N$, wobei hierin bereits bekannte Befunddaten von Vergleichspatienten gespeichert sind, bezüglich derer eine eindeutige Diagnose ("gesund" oder "krank") bekannt ist.

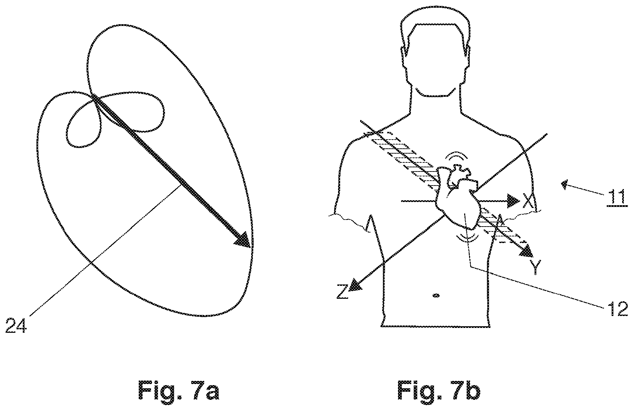

Auf Grundlage der EKG-Messdaten, die mithilfe der vier Sensoren S1-S4 am Herzen 12 eines Patienten 11 aufgenommen werden, kann durch die Auswerteeinrichtung 18 ein Raumvektor 24 erzeugt werden, der die elektrische Aktivität des Herzens 12 abbildet. Konkret bildet dieser Raumvektor 24 den zeitlichen Verlauf des Summensektors des elektrischen Feldes des Herzens 12 und weist eine der Feldrichtung entsprechende Richtung und eine dem Potenzial entsprechende Länge auf. Ein solcher Raumvektor 24 ist beispielhaft in der Fig. 7a gezeigt, der vorzugsweise in einem orthogonalen System abgebildet wird, das aus den Achsen x, y, z nach dem Sanz'schen System (gemäß EP 86 429 B1) gebildet wird. Fig. 7b zeigt nochmals - in gleicher Weise wie Fig. 4 - in vereinfachter Weise das Herz 12 eines Patienten 11, in Verbindung mit den Achsen x, y, z nach dem Sanz'schen System.

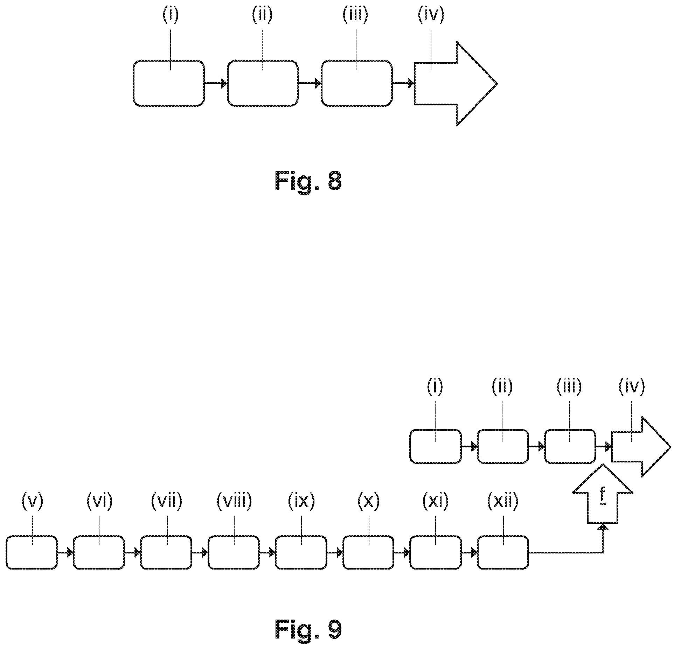

Nachstehend ist ein Verfahren nach der vorliegenden Erfindung unter Bezugnahme auf die Fig. 8 erläutert, die ein Ablaufdiagramm von Schritten eines solchen Verfahrens zeigt. Hierzu im Einzelnen:

Zu Beginn des Verfahrens werden die Sensoren S1-S4 der vorstehend erläuterten Einrichtung 10 (vgl. Fig. 1) am Oberkörper eines Patienten 11 angebracht, nämlich in Entsprechung der vier Ableitungspunkte E1, E2, E3 und E4 (vgl. Fig. 2a, Fig. 2b). Zu diesem Zweck kann das T-Shirt 22 von Fig. 3 eingesetzt werden. In dieser Weise werden dann am Herzen 12 des Patienten 11 in nicht-invasiver Weise EKG-Signale aufgenommen, nämlich im Ruhezustand des Patienten 11. Dies entspricht einem Schritt (i) des Verfahrens gemäß Fig. 8.

Im Anschluss daran werden in einem Schritt (ii) des Verfahrens von Fig. 8 die aufgenommenen EKG-Signale filtertechnisch aufbereitet, nämlich, wie unter Bezugnahme auf Fig. 1 erläutert, durch den Filter 16. Ein solches Filtern dient dazu, hochfrequentes Rauschen und niederfrequente Störungen (z.B. verursacht durch die Atmung des Patienten) zu eliminieren. Beispielhafte Filtertypen sind Kerbfilter, Hochpassfilter, Sarvitzky-Golay-Tiefpassfilter.

In dem hiernach folgenden Schritt (iii) des Verfahrens von Fig. 8 werden dann die gefilterten EKG-Signale mittels der Auswerteeinrichtung 18, die signaltechnisch an den Filter 16 angeschlossen ist, auf Grundlage der Vektorkardiographie in orthogonalisierte Messgrößen überführt. Hierbei werden signifikante Bereiche in dem Signal eines Herzschlags lokalisiert, z.B. Beginn und Ende des QRS-Komplexes, Beginn, Maximum und Ende der T-Welle. Zur Bestimmung der tatsächlichen Zeitpunkte der physiologischen Extrempunkte ist es vorteilhaft, wenn die genannten Ableitungen A, D, H, I und V, die zwischen den Ableitungspunkten E1-E4 (vgl. Fig. 2a, Fig. 2b) gewonnen werden, jeweils in Kugelkoordinaten transformiert werden.

Schließlich werden in einem weiteren Schritt (iv) des Verfahrens nach Fig. 8 die orthogonalisierten Messgrößen in das KI-System 20 bzw. ein neuronales Netz $20_N$ eingegeben. Hierbei ist zu beachten, dass das KI-System 20 auf bereits bekannten Befunddaten von Vergleichspatienten basiert, für die - wie eingangs bereits erläutert - eine eindeutige Kenntnis in Bezug auf deren Gesundheitszustand ("gesund" oder "krank") vorliegt. Auf Grundlage dessen kann dann mittels des Vektordaten-Analysators 115 und des Vektordaten-Auswerte-Speichers 116 durch einen Vergleich der eingegebenen orthogonalisierten Messgrößen mit den Befunddaten der Vergleichspatienten innerhalb des KI-Systems 20 eine Diagnose für den untersuchten Patienten 11 erstellt werden.

Für den Schritt (i) des Verfahrens von Fig. 8 empfiehlt sich, dass eine nicht-invasive Aufnahme von EKG-Signalen am Herzen 12 des Patienten 11 an genau den vier Ableitungspunkten E1-E4 erfolgt, die vorstehend unter Bezugnahme auf die Fig. 2a und Fig. 2b erläutert worden sind.

Mittels einer vorteilhaften Weiterbildung bzw. Ergänzung des Verfahrens von Fig. 8 ist es möglich, dass das KI-System 20 bzw. das neuronale Netz $20_N$ vor dem Schritt (iv) trainiert wird, nämlich durch eine Eingabe von spezifischen Lernwerten f. Dies ist nachfolgend anhand der Fig. 9 gezeigt und im Einzelnen erläutert:

Die Anzahl der spezifischen Lernwerte f, mit denen das KI-System 20 bzw. das neuronale Netz $20_N$ vor dem Schritt (iv) trainiert wird, kann zwischen 10 und 30 liegen, und z.B. den Wert von 20 annehmen. Diese spezifischen Lernwerte f werden durch folgende Schrittabfolge bestimmt:

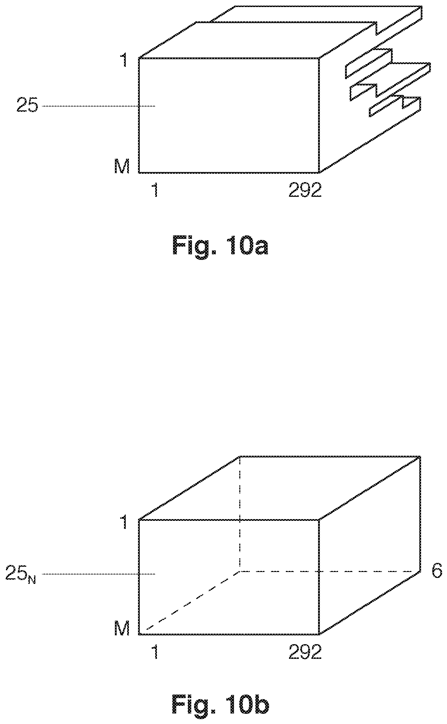

(v) Bereitstellen von Messgrößen von einer Menge M (vgl. Fig. 10) von Patienten 11 mit einem bekannten Befund, wobei diese Messgrößen auf Grundlage der Vektorkardiographie orthogonalisiert sind,

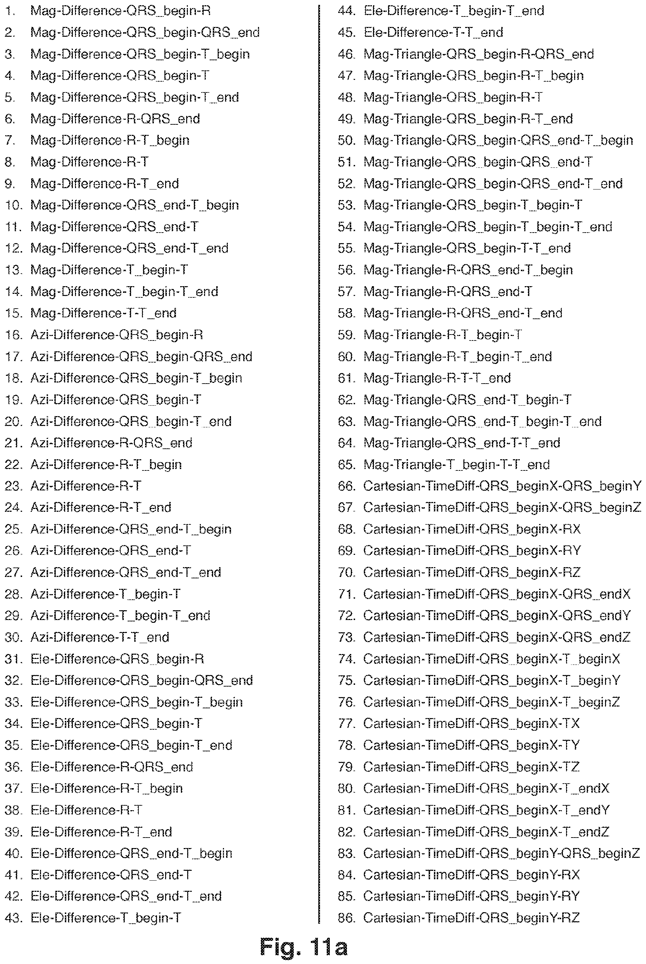

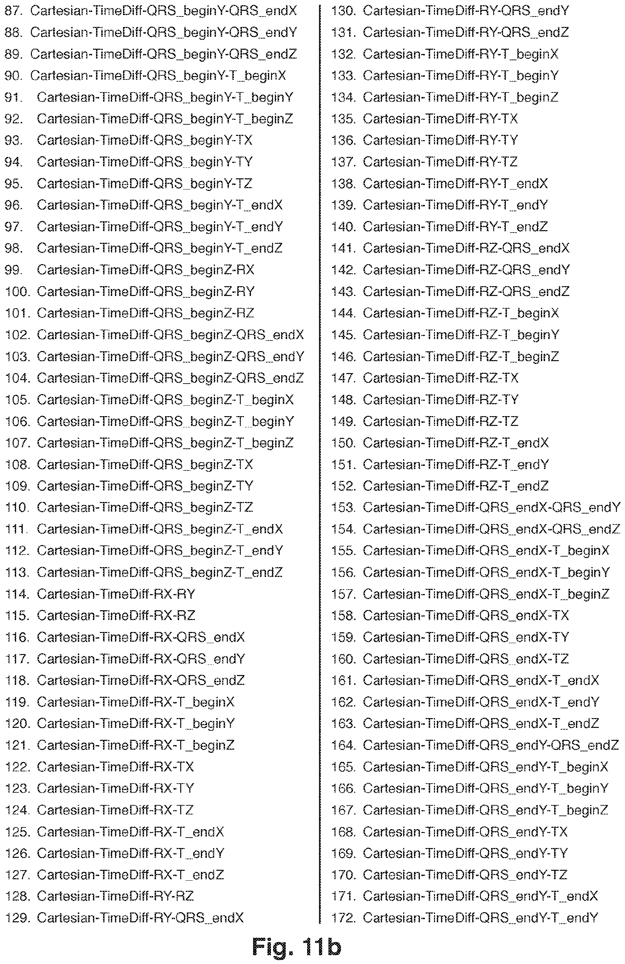

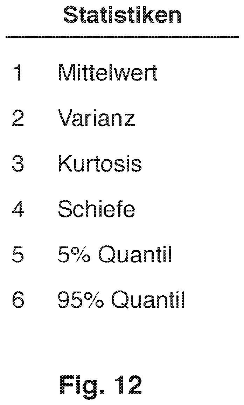

(vi) Bereitstellen einer Mehrzahl von Zeitreihen-Parametern (vgl. Fig. 11a und Fig. 11b) und zumindest einem statistischen Verfahren (vgl. Fig. 12),

(vii) Bilden einer 3D-Matrix 25 (vgl. Fig. 10a), wobei die orthogonalisierten Messgrößen der Menge (M) von Patienten die Zeilen, die Zeitreihen-Parameter die Spalten und die Zeitreihenlänge die Tiefe dieser Matrix (25) definieren, wobei im Falle skalarer Parameter die Tiefe gleich eins ist,

(viii) Klassifizieren aller Wertepaare der 3D-Matrix (25) nach dem Prinzip der "Area-under-Curve" (AUC)-Berechnung,

(ix) Auswählen eines Wertepaars aus der Menge gemäß Schritt (viii) mit dem höchsten AUC-Wert,

(x) Überprüfen eines weiteren Wertepaars aus der Menge gemäß Schritt (viii), und Auswählen dieses Wertepaars, falls hierfür ein Schwellenwert für eine Korrelation mit dem Wertepaar von Schritt (ix) betragsmäßig kleiner als 1,65/VN ist, mit N= Anzahl der Datenpunkte bzw. Parameter-Statistiken (Patienten) gemäß Schritt (vi)

(xi) Wiederholen des Schritts (x) für ein weiteres Wertepaar aus der Menge gemäß Schritt (viii), und Auswählen dieses Wertepaars, falls ein Schwellenwert für eine Korrelation mit den zuvor ausgewählten Wertepaaren jeweils betragsmäßig kleiner als 1,65>/N ist, und

(xii) Wiederholen der Schritte (ix) bis (xi) solange, bis eine vorbestimmte Anzahl von z.B. 20 Wertepaaren erreicht ist, die dann als spezifischen Lernwerte f definiert und zum Trainieren des KI-Systems (20) darin eingegeben werden.

Die vorstehend genannten Schritte (v) bis (xii) der Weiterbildung des Verfahrens gemäß Fig. 9 sind in dem zugehörigen Ablaufdiagramm jeweils vereinfacht durch Blöcke symbolisiert. Hierbei ist der Pfeil "f", der nach dem Schritt (xii) von unten in den Ablauf zwischen den Schritten (iii) und (iv) einmündet, so zu verstehen, dass die im Schritt (xii) definierten vorbestimmte Anzahl von spezifischen Lernwerten f in das KI-System 20 bzw. das neuronale Netz $20_N$ eingegeben werden.

In Bezug auf die vorteilhafte Weiterbildung des Verfahrens gemäß Fig. 9 darf erläuternd darauf hingewiesen werden, dass in Schritt (v) die Messgrößen der Menge M von Patienten 11 in Form von Zeitreihen vorzugsweise in Millisekunden oder in Form von Herzschlägen bereitgestellt werden. Die Bildung der 3D- Matrix 25 gemäß Schritt (vii) ist symbolisch in der Fig. 10a dargestellt. Die Menge M aller Patienten ist als Ordinate aufgetragen und kann, z.B. von oben nach unten gesehen, zunächst in eine Gruppe von gesunden Patienten (z.B. ohne KHK-Befund) und dann in eine Gruppe von kranken Patienten (z.B. mit KHK-Befund) unterteilt sein. Die 3D-Matrix umfasst die Menge M der insgesamt 284 Zeitreihen-, sowie der skalaren persönlichen Parameter, welche in den Fig. 11a - 11d gezeigt sind. In das Training des neuronalen Netzes gehen jedoch nur die 282 Zeitreihen Parameter ein.

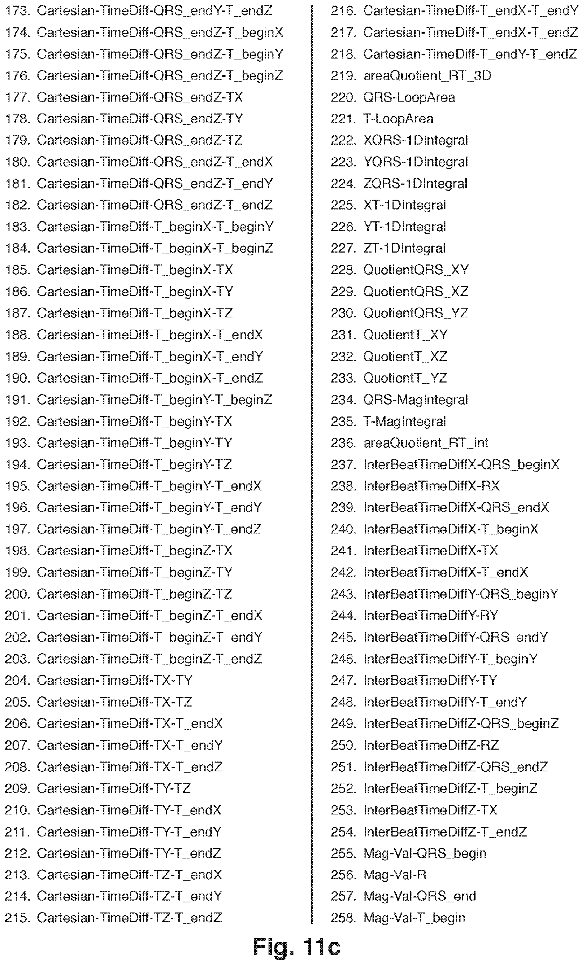

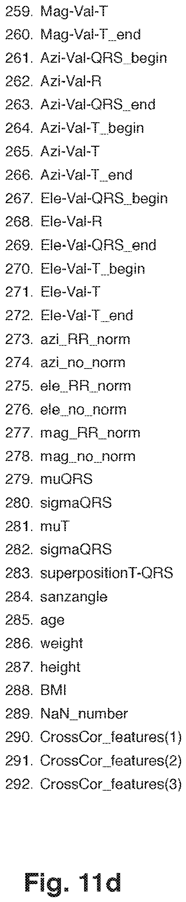

In Bezug auf die 292 Parameter, die in den Fig. 11a, 11b, 11c und 11d dargestellt sind, wird gesondert darauf hingewiesen, dass es sich hierbei um 284 Zeitreihen-Parameter handelt. 8 persönliche skalare Parameter, die sich auf einen jeweils untersuchten Patienten beziehen werden über den Satz von Bayes für die Wahrscheinlichkeitsberechnungen berücksichtigt.

An dieser Stelle wird gesondert darauf hingewiesen, dass bei dem vorstehend genannten Verfahren, in dessen Schritten (x) und (xi), die einzelnen Schwellenwerte für die jeweiligen Korrelationen mit dem Wertepaar von Schritt (ix) keinen konstanten festen Wert annehmen, sondern jeweils abhängig sind von der Anzahl von Herzschlägen bzw. von der Mehrzahl von Zeitreihen-Parametern gemäß Schritt (vi). Somit werden für kurze Zeitreihen (und damit kleinere Werte von N) höhere Korrelationen zugelassen, und umgekehrt.

Fig. 10b zeigt die 3D-Matrix in einer normierten Version mit einheitlicher Tiefe, die dadurch erreicht wird, dass deren Spalten (= Patienten-Daten) und Zeilen (= Zeitreihen-Parameter) mit einer Mehrzahl von statistischen Verfahren verknüpft bzw. verrechnet werden, von denen beispielhaft sechs Verfahren in Fig. 12 gezeigt sind, nämlich:

- Mittelwert
- Varianz
- Kurtosis,
- Schiefe,
- 5%-Quantil,
- 95%-Quantil.

Die mögliche Anwendung dieser sechs Verfahren ist in der Darstellung von Fig. 10b durch den Eintrag der "6" (im Bildbereich rechts) angedeutet. Hierzu wird darauf hingewiesen, dass im Schritt (vii) zumindest eines dieser statistischen Verfahren, oder auch mehrere solcher Verfahren, angewendet werden können, um damit sowohl die Tiefe der 3D- Matrix 25 zu definieren und ggf. eine einheitliche Tiefe zur Realisierung einer normierten Matrix $25_N$ erreicht wird.

Des Weiteren darf für das Verfahren gemäß Fig. 9 erläuternd darauf hingewiesen werden, dass in Schritt (viii) die AUC-Berechnung empirisch oder nach dem Prinzip der Johnson-Verteilung erfolgen kann. Das Prinzip der Johnson-Verteilung ist per se Stand der Technik bekannt, könnte jedoch mit der vorliegenden Erfindung erstmals im Zusammenhang mit der Auswertung von Patientendaten bzw. EKG-Signalen zwecks einer Früherkennung des Vorliegens einer KHK und/oder einer HRS eingesetzt werden.

Wie bereits erläutert, ist es zur Auswertung der Daten zwecks Erstellung einer Diagnose von Vorteil, wenn das KI-System 20, in das die spezifischen Lernwerte f eingegeben werden, zumindest ein neuronales Netz $20_N$, oder eine Mehrzahl von solchen Netzen $20_N$, aufweist.

Mittels der vorliegenden Erfindung ist es möglich, wie erläutert einerseits ein Verfahren zur Früherkennung des Vorliegens einer KHK und/oder einer HRS eines zu untersuchende Patienten 11 durchzuführen, wie es anhand des Ablaufdiagramms von Fig. 8 gezeigt und erläutert worden ist. Andererseits ist es möglich, zur Verbesserung der Diagnose des Patienten 11 das KI-System 20 (bzw. ein neuronales Netz $20_N$) vor der eigentlichen Messung einem Training zu unterziehen, wie es anhand des Ablaufdiagramms von Fig. 9 gezeigt und erläutert worden ist. Ein solches Training wird auf Grundlage der Daten von solchen Patienten durchgeführt, von denen eine eindeutige Kenntnis in Bezug auf deren Gesundheitszustand ("gesund" oder "krank") vorliegt. Insoweit ist ein solches Training im Sinne der vorigen Erfindung auch als "überwachtes Training" (*supervised learning*) zu verstehen.

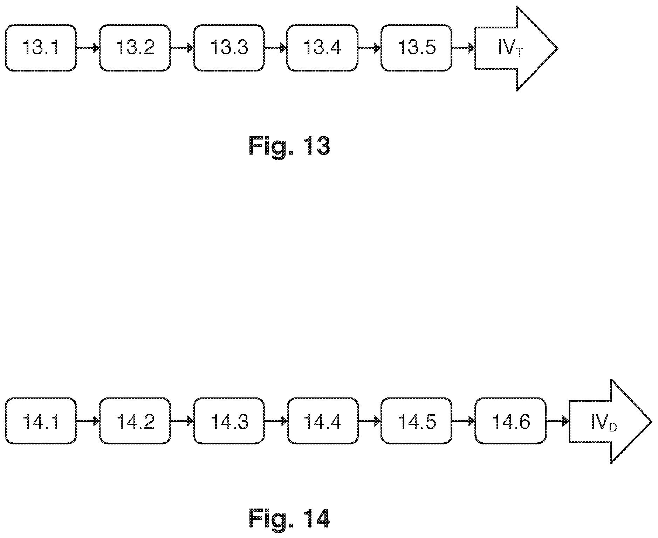

Diese beiden Möglichkeiten, nämlich sowohl ein vorgeschaltetes Training für das KI-System 20 (bzw. ein neuronales Netz $20_N$) als auch die eigentliche Durchführung der Vermessung eines Patienten 11 zwecks Erstellung einer gewünschten Diagnose, sind nachfolgend nochmals in den Ablaufdiagrammen der Fig. 13 und Fig. 14 dargestellt. Das Ablaufdiagramm von Fig. 13 zeigt mit seinen Blöcken 13.1 - 13.5 eine Schrittabfolge zwecks eines Trainings des KI-Systems 20. Ein solches Training dient zur Optimierung der eigentlichen Untersuchung bzw. Diagnose eines Patienten 11, die durch die Schrittabfolge des Ablaufdiagramms von Fig. 14 veranschaulicht ist.

Im Ablaufdiagramm von Fig. 13 entspricht der Schritt 13.1 im Wesentlichen dem Schritt (i) von Fig. 8, wobei der Schritt 13.2 im Wesentlichen dem Schritt (ii) von Fig. 8 entspricht. Gleiches gilt auch für den Schritt 13.3, der im Wesentlichen dem Schritt (iii) von Fig. 8 entspricht. Zur Vermeidung von Wiederholungen darf deshalb für diese Schritte 13.1, 13.2 und 13.3 auf die Erläuterungen zu Fig. 8 verwiesen werden.

Der nun folgende Schritt 13.4 im Ablaufdiagramm von Fig. 13 entspricht einer Parameter-Gewinnung ("*parameter extraction*"), bei der - in Entsprechung der Schritte (v) bis (vii) von Fig. 9 - Zeitreihen-Parameter mit den Messgrößen einer Menge M von Patienten 11 verknüpft bzw. verrechnet werden. Auch hierbei erweist es sich als Vorteil, wenn dies auf Grundlage von Kugelkoordinaten erfolgt, in welche die Ableitungen A, D, H, I und V geeignet transformiert worden sind.

Der anschließende Schritt 13.5 bezweckt eine Merkmals-Bestimmung (*"feature evaluation"*) und entspricht im Wesentlichen eine Abfolge der Schritte (viii) bis (xii) von Fig. 9, die vorstehend bereits genannt und erläutert worden sind. Dies bedeutet, dass mit dieser Merkmals-Bestimmung gemäß Schritt 13.5 die spezifischen Lernwerte f bestimmt bzw. ermittelt werden.

Im Anschluss daran werden dann bei dem Ablaufdiagramm von Fig. 13, nämlich im Schritt $IV_T$, die spezifischen Lernwerte f in das KI-System 20 (bzw. in ein neuronales Netz $20_N$) eingegeben, in Entsprechung des Schritts (iv) von Fig. 8. Im Ergebnis wird damit durch die Eingabe der spezifischen Lernwerte f das KI-System 20 geeignet trainiert. An dieser Stelle darf nochmals darauf hingewiesen werden, dass es im Rahmen dieses Trainings von großem Vorteil ist, dass die Anzahl der spezifischen Lernwerte f relativ gering ist, und z.B. den Wert 20 annehmen kann. Alternativ hierzu kann die Anzahl der spezifischen Lernwerte f auch kleiner oder größer 20 sein, und z.B. 15 oder 25 betragen. Jedenfalls ist in Bezug auf das hier diskutierte Training des KI-Systems 20 mit Hilfe dieser Lernwerte f zu verstehen, dass diese stets anhand der Daten eines Patienten 11 gewonnen werden, für den eine eindeutige Kenntnis in Bezug auf deren Gesundheitszustand ("gesund" oder "krank") vorliegt.

Das Ablaufdiagramm von Fig. 14 veranschaulicht mit seiner Schrittabfolge dann die tatsächlich durchgeführte Diagnose für einen Patienten 11. Hierbei entsprechen die Schritte 14.1, 14.2 und 14.3 im Wesentlichen den Schritten 13.1-13.3, so dass zur Vermeidung von Wiederholungen auf die Erläuterung zu den Schritten 13.1-13.3 verwiesen werden darf.

Der Schritt 14.5 beim Ablaufdiagramm von Fig. 14 sieht eine Merkmals-Auswahl (*"feature selection"*) vor - hierbei werden nur die Daten eines untersuchten Patienten 11 auf Grundlage der aufgenommenen EKG-Signale verwendet, die den zuvor gemäß Schritt 13.5 bestimmten spezifischen Lernwerten f entsprechen. Ein Vergleich dieser ausgewählten Merkmale bzw. Werte mit den vorbestimmten spezifischen Lernwerten f erfolgt dann in Schritt 14.6 ("trained network"). Im Anschluss hieran wird dann im Schritt $IV_D$ mit Hilfe des trainierten KI-Systems 20 für den Patienten 11 die eigentliche Diagnose $IV_D$ gestellt, in Entsprechung des Schritts (iv) von Fig. 8.

Die vorstehend erläuterte Diagnose eines Patienten 11, die mit einer Einrichtung 10 von Fig. 1 und durch ein Verfahren nach dem Ablaufdiagramm von Fig. 8 bzw. Fig. 14 durchgeführt wird, als auch das Training eines KI-Systems 20 (bzw. eines neuronalen Netzes 20N), das nach dem Ablaufdiagramm von Fig. 9 bzw. Fig. 13 durchgeführt wird, beruhen stets darauf, dass die Sensoren S1-S4 an dem Körper des Patienten 11 angebracht werden, z.B. unter Verwendung des T-Shirts von Fig. 3. Hierbei ist es nun nach einem weiteren Verfahren gemäß der vorliegenden Erfindung möglich, einen ggf. nicht korrekten Sitz dieser Sensoren S1-S4 am Körper des Patienten 11 zu kompensieren, so dass damit eine weiterhin realistische Diagnose für den Patienten 11 gewährleistet ist. Ein solches Verfahren, nachstehend kurz als "Korrektur-Verfahren" bezeichnet, sieht zunächst vor, dass ein auf das Herz 12 des Patienten 11 bezogener Raumvektor 24 (vgl. Fig. 7a), der den Vektor des durch die Aktivität des Herzens 12 gebildeten elektrischen Feldes darstellt, ermittelt und geeignet dargestellt wird. Hierbei werden Messwerte des Herzens 12 am Körper 14 des Patienten an den insgesamt vier Ableitungspunkten E1-E4 erfasst, wie vorstehend bereits im Zusammenhang mit der Fig. 2 erläutert worden ist. Bei dem soeben genannten Korrektur-Verfahren wird auf Grundlage der Ableitungen A, D, H, I und V (vgl. Fig. 5a, Fig. 5b) ein orthogonales System mit den Beziehungen

$$x = D \cos 45° - I$$

$$y = D \sin 45° + A$$

$$z = (V - H) \sin 45°$$

gebildet, wobei die Messwerte für den Patienten 11 und der daraus ermittelte Raumvektor 24 in diesem orthogonalen System x, y, z abgebildet werden.

Damit sieht dann das Korrektur-Verfahren unter Bezugnahme auf die Darstellungen in den Fig. 5a, Fig. 5b, Fig. 15 und Fig. 16 im Einzelnen folgende Schritte vor:

(a) Durchführung einer Messung an einem Patienten unter Verwendung der ersten bis vierten Ableitungspunkte E1-E4 am Körper 14 des Patienten, um damit für diesen Patienten ein Kardiogramm zu gewinnen,

(b) Extrahieren der Amplituden der R-Welle aus dem Kardiogramm von Schritt (a) für jeden Herzschlag in x-, y- und z-Richtung,

(c) Ermitteln von Mittelwerten $\mu_x$, $\mu_y$, $\mu_z$ und Standard-Abweichungen $\sigma_x$, $\sigma_y$, $\sigma_z$ der jeweils in Millivolt erfassten Amplituden aus dem Kardiogramm gemäß Schritt (b), wobei mit diesen Mittelwerten $\mu_x$, $\mu_y$, $\mu_z$ und Standard-Abweichungen $\sigma_x$, $\sigma_y$, $\sigma_z$ dann ein Berechnungsvektor 26 gebildet wird,

(d) Bilden einer Koeffizientenmatrix 28, die auf Grundlage einer Hauptachsentransformation für Messungen an Vergleichs-Patienten mit unterschiedlichen Anlageschemata gewonnen worden ist,

(e) Multiplizieren des Berechnungsvektors 26 von Schritt (c) mit der Koeffizientenmatrix 28 von Schritt (d), zur Bildung eines Ergebnisvektors 30 mit insgesamt sechs Hauptachsen $PC_1$ - $PC_6$,

(f) Extrahieren der ersten Hauptachse $PC_1$ und der zweiten Hauptachse $PC_2$ aus dem Ergebnisvektor 30 von Schritt (e), zur Bildung eines Referenzpunktes $PC_1$, $PC_2$ im Raum der ersten und zweiten Hauptachse,

(g) Ermitteln eines euklidischen Abstands des Referenzpunktes $PC_1$, $PC_2$ von einem vorbestimmten Zielpunkt $PC_1^{fit}$, $PC_2^{fit}$, der einer korrekten Position der vier Ableitungspunkte E1-E4 am menschlichen Körper 14 entspricht, und

(h) falls der Abstand des Referenzpunktes $PC_1$, $PC_2$ von dem vorbestimmten Zielpunkt $PC_1^{fit}$, $PC_2^{fit}$ größer als ein vorbestimmter Maximalwert ist: Durchführen einer Winkelkorrektur für ein aus dem ersten Ableitungspunkt E1, dem dritten Ableitungspunkt E3 und dem vierten Ableitungspunkt E4 gebildetes erstes Dreieck 31 und für ein aus dem ersten Ableitungspunkt E1, dem zweiten Ableitungspunkt E2 und dem vierten Ableitungspunkt E4 gebildetes zweites Dreieck 32, so dass damit der euklidische Abstand zwischen dem Referenzpunkt $PC_1$, $PC_2$ und dem vorbestimmten Zielpunkt $PC_1^{fit}$, $PC_2^{fit}$ durch Anpassung des orthogonalen Systems x, y, z an die geänderte Geometrie minimiert wird.

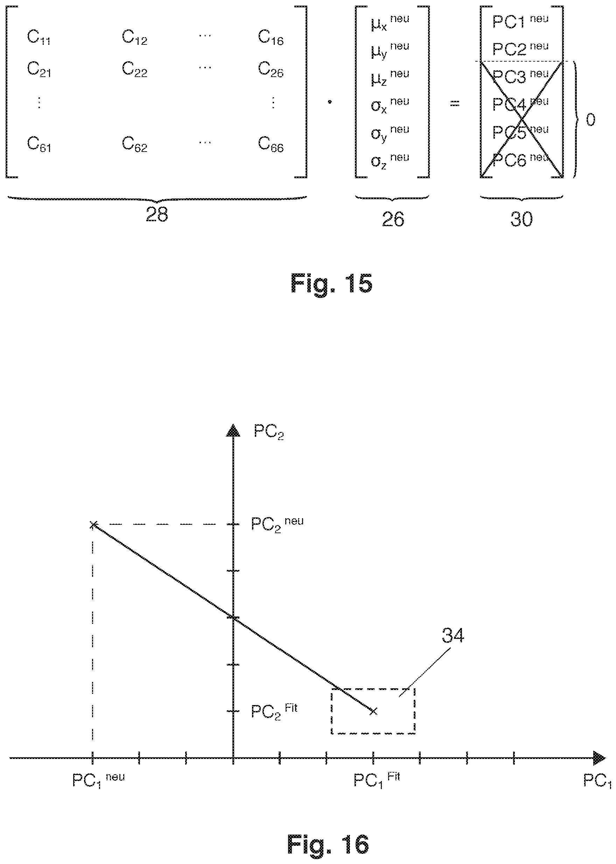

Die obigen Schritte (a) bis (h) des Korrektur-Verfahrens sind dahingehend erläutert, dass es sich bei den Amplituden, mit denen im Schritt (c) der Berechnungsvektor 26 gebildet wird, um jene Messwerte handelt, die zuvor durch die Messung gemäß Schritt (a) bzw. bei dem Verfahren von Fig. 8 in dessen Schritt (i), beim Verfahren von Fig. 13 in dessen Schritt 13.1 und/oder beim Verfahren von Fig. 14 in dessen Schritt 14.1 nicht-invasiv mit den EKG-Signalen am Herzen 12 des Patienten 11 aufgenommen werden. Der Schritt (e), wonach der Berechnungsvektor 26 mit der 6x6-Koeffizientenmatrix 28 von Schritt (d) multipliziert und daraus der Ergebnisvektor 30 resultiert, ist in der Fig. 15 veranschaulicht. Dieser Ergebnisvektor 30 liegt in Form einer 6x1-Matrix vor. Im Schritt (f) werden die dritte bis sechste Zeile des Ergebnisvektors 30, wie in Fig. 15 mit der Durchstreichung kenntlich gemacht, gestrichen bzw. für die weitere Berechnung ignoriert, womit die erste Hauptachse $PC_1$ und die zweite Hauptachse $PC_2$ aus dem Ergebnisvektor 30 extrahiert werden, zur Bildung des Referenzpunktes $PC_1$, $PC_2$. Des Weiteren ist der euklidische Abstand, der im Schritt (g) des Korrektur-Verfahrens ermittelt wird, ist in dem Diagramm von Fig. 16 veranschaulicht.

Das Diagramm von Fig. 16 zeigt auch ein beispielhaftes Zielgebiet, hier durch ein Rechteck 34 mit gestrichelten Linien symbolisiert. Diesbezüglich wird darauf hingewiesen, dass vor Durchführung des Schritts (h) der vorbestimmte Zielpunkt $PC_1^{fit}$, $PC_2^{fit}$ aus diesem Zielgebiet ausgewählt wird, das auf Grundlage von Standard-Abweichungen einer Mehrzahl von überprüften korrekten Messungen des Zielpunktes am menschlichen Körper 14 gebildet wird.

Im Schritt (h) des Korrektur-Verfahrens können durch die hierin definierte Winkelkorrektur Anpassungswerte $\varepsilon$, $\eta$ ermittelt werden, mit denen die nicht-rechtwinkligen Winkel $\alpha$ des ersten Dreiecks 31 und die nicht-rechtwinkligen Winkel $\beta$ des zweiten Dreiecks 32 korrigiert werden können. Damit ist es möglich, eine insbesondere nicht korrekte Position des ersten Ableitungspunkts E1 am menschlichen Körper 14 zu kompensieren, um gegebenenfalls nicht-rechtwinklige Dreiecke zu berücksichtigen. In dieser Weise stellt der Anpassungswert $\varepsilon$ einen adjustierten Wert dar, der im Falle von korrekt angelegten Elektroden identisch mit dem Winkel $\alpha$ des ersten Dreiecks 31 ist. Gleiches gilt für den adjustierten Wert $\eta$, der im Falle von korrekt angelegten Elektroden mit dem Winkel $\beta$ des zweiten Dreiecks 32 übereinstimmt. Dieser Zusammenhang ist auch grafisch in den Darstellungen von Fig. 5a und Fig. 5b gezeigt.

Die vorstehend genannten Anpassungswerte $\varepsilon$, $\eta$, die für die Winkelkorrektur von Schritt (e) verwendet werden können, lassen sich durch Minimierung des euklidischen Abstands zwischen Referenz und Zielpunkt bestimmen.

Die Koeffizientenmatrix 28, mit der in Schritt (e) der Berechnungsvektor 26 multipliziert wird, wird über eine Hauptachsentransformation einer 23x6-Matrix gebildet, die auf 23 Testmessungen und den Mittelwerten und Standard-Abweichungen der daraus ermittelten Messwerte beruht.

Das vorstehend erläuterte Korrektur-Verfahren beruht auf dem Prinzip einer Hauptachsen-Transformation, mit der im Ergebnis ein nicht korrekter Sitz von Elektroden bzw. der Sensoren S1-S4 am menschlichen Körper 14 kompensiert werden kann. Dies gilt insbesondere für die Position des Sensors S1, der dem ersten Ableitungspunkt E1 zugeordnet ist, und ist z.B. für den Fall vorteilhaft, dass die Messwerte des Herzens 12 mit dem T-Shirt 22 von Fig. 3 erfasst werden.

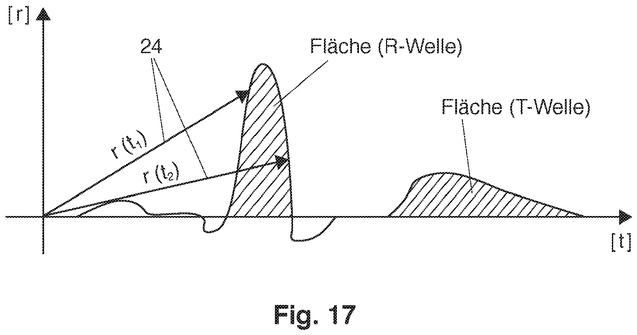

Vorstehend ist bei der Diskussion von Fig. 7a bereits darauf hingewiesen worden, dass der darin gezeigte Raumvektor 24, der anhand der Vektorkardiographie bestimmt wird, die elektrische Aktivität des Herzens 12 darstellt. Für die vorliegende Erfindung wird damit auch ein Herzüberwachungsverfahren definiert, bei dem ein Quotient aus den Flächen, die von einer Länge des Raumvektors 24 (= Radiusvektor) als Funktion der Zeit jeweils während der R-Welle und während der T-Welle überstrichen werden, gebildet wird, wobei anschließend dieser Quotient einer weiteren Auswertung zugeführt wird. Diese von dem Raumvektor 24 als Funktion der Zeit überstrichenen Flächen sind beispielhaft in dem Diagramm von Fig. 17 gezeigt.

Für die vorliegende Erfindung hat sich herausgestellt, dass für das untersuchte Herz 12 eines Patienten 11 eine Ischämie bzw. eine koronare Herzkrankheit (KHK) erkannt wird, falls der aus den von dem Raumvektor 24 jeweils während der R-Welle und während der T-Welle überstrichenen Flächen gebildete Quotient

$$\frac{\text{Fläche (R-Welle)}}{\text{Fläche (T-Welle)}} = a$$

außerhalb eines Intervalls zwischen den Grenzwerten $a_{0,KHK}$ und $a_{1,KHK}$ liegt.

Einen Sonderfall der vorstehend genannten Erkenntnisse bildet der Fall, dass für das untersuchte Herz 12 eine Herzrhythmusstörung (HRS) erkannt wird, falls der aus den von dem Raumvektor 24 jeweils während der R-Welle und während der T-Welle überstrichenen Flächen gebildete Quotient die Bedingung

$$\frac{\text{Fläche (R-Welle)}}{\text{Fläche (T-Welle)}} < a_{0,\ HRS}$$

oder die Bedingung

$$\frac{\text{Fläche (R-Welle)}}{\text{Fläche (T-Welle)}} > a_{1,\ HRS}$$

erfüllt.

Hinsichtlich der Grenzwerte $a_{0,KHK}$ und $a_{1,KHK}$ bzw $a_{0,HRS}$ und $a_{1,\ HRS}$, mit denen der Quotient gebildet aus der Fläche (R-Welle/Fläche (T-Welle) bei der Untersuchung des Vorliegens einer KHK und/oder HRS bei der tatsächlichen Untersuchung eines Patienten jeweils verglichen bzw. korreliert wird, darf zur Vermeidung von Wiederholungen auf die Erläuterungen im einleitenden Teil der vorliegenden Patentanmeldung verwiesen werden, wonach diese Grenzwerte auch in Abhängigkeit von einem Trainingsset bestimmt bzw. optimiert werden können.

Die verschiedenen Erkenntnisse, die für das zuletzt genannte Verfahren nach der vorliegenden Erfindung auf dem Verhältnis der Flächen, die von dem Raumvektor 24 als Funktion der Zeit jeweils während der R-Welle und während der T-Welle überstrichen werden, können selbstverständlich auch bei den zuvor genannten erfindungsgemäßen Verfahren angewendet bzw. berücksichtigt werden, die anhand der Ablaufdiagramme nach Fig. 8, Fig. 9, Fig. 13 bzw. Fig. 14 gezeigt und erläutert worden sind.

Bezugszeichenliste

| 10 | Einrichtung zur Früherkennung einer KHK oder einer HRS |
| 11 | Patient |
| 12 | Herz (des Patienten 11) |
| 14 | Körper (des Patienten 11) |
| 16 | Filter |
| 18 | Auswerteeinrichtung |
| 20 | System basierend auf Künstlicher Intelligenz (KI) |
| $20_N$ | Neuronales Netz |
| 22 | T-Shirt |
| 24 | Raumvektor |
| 25 | 3D-Matrix |
| $25_N$ | normierte 3D-Matrix |
| 26 | Berechnungsvektor |
| 28 | Koeffizientenmatrix |
| 30 | Ergebnisvektor |
| 31 | erstes Dreieck |
| 32 | zweites Dreieck |
| 34 | Zielgebiet |
| 13.1-13.5 | Schritte eines erfindungsgemäßen Verfahrens (vgl. Fig. 13) |
| 14.1-14.5 | Schritte eines erfindungsgemäßen Verfahrens (vgl. Fig. 14) |
| 100 | Sensoreinrichtung |
| 102 | Datenaufzeichner |
| 103 | Signalempfänger |
| 104 | Signalkonverter |
| 105 | Signalspeicher |
| 106 | Vorrichtung |
| 107 | Signalleser |
| 108 | Vektordaten-Generator |
| 109 | Vektordaten-Speicher |
| 110 | Vektordaten-Auswerter |
| 111 | Vektordaten-Synchronisierer |
| 112 | Vektordaten-Anzeige |
| 113 | Server |
| 114 | Vektordaten-Speicher |
| 115 | Vektordaten-Analysator |
| 116 | Vektordaten-Auswerte-Speicher |
| A | anteriore Ableitung |
| D | dorsale Ableitung |
| E1 | erste Ableitungspunkt |
| E2 | zweiter Ableitungspunkt |
| E3 | dritter Ableitungspunkt |
| E4 | vierte Ableitungspunkt |
| f | spezifische Lernwerte (für das neuronale Netz 26) |
| H | horizontale Ableitung |

| | |
|---|---|
| I | inferiore Ableitung |
| M | Menge an Patienten (mit bekanntem Befund) |
| $PC_1$ | erste Hauptachse |
| $PC_2$ | zweite Hauptachse |
| S1-S4 | Sensoren, die den Ableitungspunkten E1-E4 zugeordnet sind |
| V | vertikale Ableitung |
| $\alpha$ | Nicht-rechtwinklige Winkel des ersten Dreiecks 21 (E1-E3-E4) |
| $\beta$ | Nicht-rechtwinklige Winkel des zweiten Dreiecks 22 (E1-E2-E4) |
| $\varepsilon, \eta$ | Anpassungswerte für die Winkelkorrektur, für die Winkel $\alpha$ und $\beta$ |

**Patentansprüche**

1. Verfahren zur Früherkennung des Vorliegens einer koronaren Herzkrankheit (KHK) und/oder einer Herzrhythmusstörung (HRS) eines zu untersuchenden Patienten (11), mit den Schritten:

   (i) nicht-invasive Aufnahme von EKG-Signalen am Herzen (12) des Patienten (11) in dessen Ruhezustand,
   (ii) filtertechnische Aufbereitung der aufgenommenen EKG-Signale,
   (iii) Überführung der gefilterten EKG-Signale in orthogonalisierte Messgrößen auf Grundlage der Vektorkardiographie,
   **gekennzeichnet durch**
   (iv) Eingabe der orthogonalisierten Messgrößen in ein System (20) basierend auf Künstlicher Intelligenz (KI), in dem bereits bekannte Befunddaten von Vergleichspatienten gespeichert sind, wobei **durch** einen Vergleich der eingegebenen orthogonalisierten Messgrößen mit den Befunddaten der Vergleichspatienten innerhalb des KI-Systems (20) eine Diagnose für den untersuchten Patienten (11) erstellt wird,
   und weiter **dadurch** gekennzeichnet,
   dass das KI-System (20) vor dem Schritt (iv) trainiert wird, wobei das KI-System (20) zumindest ein neuronales Netz ($20_N$) aufweist,
   dass zum Trainieren des KI-Systems (20) darin eine Anzahl von spezifischen Lernwerten (f) eingegeben wird, vorzugsweise, dass die Anzahl von spezifischen Lernwerten (f) zwischen 10 und 30 beträgt, weiter vorzugsweise, dass die Anzahl von spezifischen Lernwerten (f) 20 beträgt, und dass die spezifischen Lernwerte (f) **durch** folgende Schrittabfolge bestimmt werden:

   (v) Bereitstellen von Messgrößen von einer Menge (M) von Patienten mit einem bekannten Befund, wobei diese Messgrößen auf Grundlage der Vektorkardiographie orthogonalisiert sind,
   (vi) Bereitstellen einer Mehrzahl von Zeitreihen-Parametern und zumindest einer Statistik,
   (vii) Bilden einer 3D-Matrix (25), wobei die orthogonalisierten Messgrößen der Menge (M) von Patienten die Zeilen, die Zeitreihen-Parameter die Spalten und das zumindest eine statistische Verfahren die Tiefe dieser Matrix (25) definieren,
   (viii) Klassifizieren aller Wertepaare der 3D-Matrix (25) nach dem Prinzip der "Area-under-Curve" (AUC)-Berechnung,
   (ix) Auswählen eines Wertepaars aus der Menge gemäß Schritt (viii) mit dem höchsten AUC-Wert,
   (x) Überprüfen eines weiteren Wertepaars aus der Menge gemäß Schritt (viii), und Auswählen dieses Wertepaars, falls hierfür ein Schwellenwert für eine Korrelation mit dem Wertepaar von Schritt (ix) betragsmäßig kleiner als $1,65/\sqrt{N}$ ist, mit N = Anzahl der Datenpunkte bzw. Parameter-Statistiken (Patienten) gemäß Schritt (vi),
   (xi) Wiederholen des Schritts (x) für ein weiteres Wertepaar aus der Menge gemäß Schritt (viii), und Auswählen dieses Wertepaars, falls ein Schwellenwert für eine Korrelation mit den zuvor ausgewählten Wertepaaren jeweils betragsmäßig kleiner als $1,65/\sqrt{N}$ ist, und
   (xii) Wiederholen der Schritte (ix) bis (xi) solange, bis eine vorbestimmte Anzahl von Wertepaaren erreicht ist, die dann als spezifischen Lernwerte (f) zum Trainieren des KI-Systems (20) definiert werden.

2. Verfahren nach Anspruch 1, **dadurch gekennzeichnet, dass** in Schritt (i) die EKG-Signale an insgesamt vier Ableitungspunkten (E1-E4) am Körper (14) des Patienten (11) aufgenommen werden.

3. Verfahren nach Anspruch 2, **dadurch gekennzeichnet, dass** Potentialdifferenzen in Form einer anterioren Ableitung (A) zwischen einem ersten Ableitungspunkt (E1) und einem vierten Ableitungspunkt (E4), einer dorsalen Ableitung (D) zwischen einem zweiten Ableitungspunkt (E2) und dem vierten Ableitungspunkt (E4), einer horizontalen Ableitung (H) zwischen einem dritten Ableitungspunkt (E3) und dem vierten Ableitungspunkt (E4), einer vertikalen Ableitung (V) zwischen dem ersten Ableitungspunkt (E1) und dem dritten Ableitungspunkt (E3), und einer inferioren Ableitung (I) zwischen dem ersten Ableitungspunkt (E1) und dem zweiten Ableitungspunkt (E2) gemessen werden.

4. Verfahren nach Anspruch 3, **dadurch gekennzeichnet, dass** die Ableitungen (A, D, H, I, V) zwischen den jeweiligen Ableitungspunkten (E1-E4) in Kugelkoordinaten transformiert werden.

5. Verfahren nach einem der Ansprüche 2 bis 4, **dadurch gekennzeichnet, dass** zur Aufnahme der EKG-Signale ein T-Shirt (22) verwendet wird, das vier Sensoren (S1-S4), die einer korrekten Position der vier Ableitungspunkte (E1-E4) am Körper (14) des Patienten (11) zugeordnet sind, aufweist.

6. Verfahren nach einem der vorhergehenden Ansprüche, **dadurch gekennzeichnet, dass** in Schritt (v) die Messgrößen der Menge (M) von Patienten (11) in Form von Zeitreihen vorzugsweise in Millisekunden oder in Form von Herzschlägen bereitgestellt werden.

7. Verfahren nach einem der vorhergehenden Ansprüche, **dadurch gekennzeichnet, dass** in Schritt (vi) eine Mehrzahl von statistischen Verfahren (Mittelwert, Varianz, Kurtosis, Schiefe, 5%-Quantil, 95%-Quantil) bereitgestellt werden.

8. Verfahren nach Anspruch 7, **dadurch gekennzeichnet, dass** nach dem Schritt (vii) aus den Daten der 3D-Matrix (25) über ein statistisches Verfahren die normierte Matrix ($25_N$) berechnet und damit eine einheitliche Tiefe erreicht wird.

9. Verfahren nach einem der vorhergehenden Ansprüche, **dadurch gekennzeichnet, dass** in Schritt (viii) die AUC-Berechnung empirisch oder nach dem Prinzip der Johnson-Verteilung erfolgt.

10. Einrichtung (10) zur Früherkennung des Vorliegens einer koronaren Herzkrankheit (KHK) und/oder einer Herzrhythmusstörung (HRS) eines zu untersuchenden Patienten (11), umfassend eine Mehrzahl von Sensoren (S1-S4), die am Körper (14) des zu untersuchenden Patienten (11) an vorbestimmten Ableitungspunkten (E1-E4) positionierbar sind, um damit nicht-invasiv EKG-Signale am Herzen (12) des Patienten (11) vorzugsweise in dessen Ruhezustand aufzunehmen,

mindestens einen Filter (16), mit dem die aufgenommenen EKG-Signale filterbar sind,
eine Auswerteeinrichtung (18), mit der die gefilterten EKG-Signale auf Grundlage der Vektorkardiographie in orthogonalisierte Messgrößen überführbar sind, und
ein System (20) basierend auf Künstlicher Intelligenz (KI), in dem bereits bekannte Befunddaten von Vergleichspatienten gespeichert sind, wobei die orthogonalisierten Messgrößen in das K!-System (20) eingebbar und darin mit den Befunddaten der Vergleichspatienten vergleichbar sind, um damit eine Diagnose für den untersuchten Patienten (11) zu erstellen,
wobei insgesamt vier Sensoren (S1, S2, S3, S4) vorgesehen sind, in Entsprechung von insgesamt vier Ableitungspunkten (E1-E4) am Körper (14) des Patienten (11), und
wobei eine Position zumindest des Sensors (S1), der dem ersten Ableitungspunkt (E1) zugeordnet ist, mittels der Einrichtung (10) überprüfbar ist und hierzu die Einrichtung (10) programmtechnisch derart eingerichtet ist, dass ein auf das Herz (12) bezogener Raumvektor (24) ermittelbar und darstellbar ist, wobei dieser Raumvektor (24) den Vektor des elektrischen Feldes darstellt, das durch die Aktivität des Herzens (12) gebildet wird, wobei Messwerte des Herzens (12) am Körper (14) an einem ersten Ableitungspunkt (E1), an einem zweiten Ableitungspunkt (E2), an einem dritten Ableitungspunkt (E3) und an einem vierten Ableitungspunkt (E4) erfassbar sind, wobei Potentialdifferenzen in Form einer anterioren Ableitung (A) zwischen dem ersten Ableitungspunkt (E1) und dem vierten Ableitungspunkt (E4), einer dorsalen Ableitung (D) zwischen dem zweiten Ableitungspunkt (E2) und dem vierten Ableitungspunkt (E4), einer horizontalen Ableitung (H) zwischen dem dritten Ableitungspunkt (E3) und dem vierten Ableitungspunkt (E4), einer vertikalen Ableitung (V) zwischen dem ersten Ableitungspunkt (E1) und dem dritten Ableitungspunkt (E3), und einer inferioren Ableitung (I) zwischen dem ersten Ableitungspunkt (E1) und dem zweiten Ableitungspunkt (E2) messbar sind, wobei ein orthogonales System mit den Beziehungen:

$$x = D \cos 45° - I$$

$$y = D \sin 45° + A$$

$$z = (V - H) \sin 45°$$

bildbar ist und die Messwerte und der daraus ermittelte Raumvektor (24) in diesem orthogonalen System (x, y, z) abgebildet werden,

wobei von der Einrichtung (10) folgende Schrittabfolge durchführbar ist:

(a) Durchführung einer Messung an einem Patienten unter Verwendung der ersten bis vierten Ableitungspunkte (E1-E4) am Körper (14) des Patienten, um damit für diesen Patienten ein Kardiogramm zu gewinnen,

(b) Extrahieren der Amplituden der R-Welle aus dem Kardiogramm von Schritt (a) für jeden Herzschlag in x-, y- und z-Richtung,

(c) Ermitteln von Mittelwerten $\mu_x$, $\mu_y$, $\mu_z$ und Standard-Abweichungen $\sigma_x$, $\sigma_y$, $\sigma_z$ der jeweils in Millivolt erfassten Amplituden aus dem Kardiogramm gemäß Schritt (b), wobei mit diesen Mittelwerten und Standard-Abweichungen dann ein Berechnungsvektor (26) gebildet wird,

(d) Bilden einer Koeffizientenmatrix (28), die auf Grundlage einer Hauptachsentransformation für Messungen an Vergleichs-Patienten mit unterschiedlichen Anlageschemata gewonnen worden ist,

(e) Multiplizieren des Berechnungsvektors (26) von Schritt (c) mit der Koeffizientenmatrix (28) von Schritt (d), zur Bildung eines Ergebnisvektors (30) mit insgesamt sechs Hauptachsen ($PC_1$ - $PC_6$), wobei es sich bei der Koeffizientenmatrix (28), mit welcher in Schritt (e) der Berechnungsvektor (26) zur Bildung des Ergebnisvektors (30) multipliziert wird, um eine 6x6-Koeffizientenmatrix handelt,

(f) Extrahieren der ersten Hauptachse ($PC_1$) und der zweiten Hauptachse ($PC_2$) aus dem Ergebnisvektor (30) von Schritt (e), zur Bildung eines Referenzpunktes ($PC_1$, $PC_2$) im Raum der ersten und zweiten Hauptachse,

(g) Ermitteln eines euklidischen Abstands des Referenzpunktes ($PC_1$, $PC_2$) von einem vorbestimmten Zielpunkt ($PC_1^{fit}$, $PC_2^{fit}$), der einer korrekten Position der vier Ableitungspunkte (E1-E4) am menschlichen Körper (14) entspricht, und

(h) falls der Abstand des Referenzpunktes ($PC_1$, $PC_2$) von dem vorbestimmten Zielpunkt ($PC_1^{fit}$, $PC_2^{fit}$) größer als ein vorbestimmter Maximalwert ist: Durchführen einer Winkelkorrektur für ein aus dem ersten Ableitungspunkt (E1), dem dritten Ableitungspunkt (E3) und dem vierten Ableitungspunkt (E4) gebildetes erstes Dreieck (31) und für ein aus dem ersten Ableitungspunkt (E1), dem zweiten Ableitungspunkt (E2) und dem vierten Ableitungspunkt (E4) gebildetes zweites Dreieck (32), so dass damit der euklidische Abstand zwischen dem Referenzpunkt ($PC_1$, $PC_2$) und dem vorbestimmten Zielpunkt ($PC_1^{fit}$, $PC_2^{fit}$) durch Anpassung des orthogonalen Systems (x, y, z) an die geänderte Geometrie minimiert wird, wobei in Schritt (h) durch die Winkelkorrektur Anpassungswerte ($\varepsilon$, $\eta$) ermittelt werden, mit denen die nicht-rechtwinkligen Winkel ($\alpha$) des ersten Dreiecks (31) und die nicht-rechtwinkligen Winkel ($\beta$) des zweiten Dreiecks (32) korrigiert werden, so dass dadurch zumindest eine nicht korrekte Position des ersten Ableitungspunkts (E1) am menschlichen Körper (14) kompensiert wird.

11. Einrichtung (10) nach Anspruch 10, **dadurch gekennzeichnet, dass** die Auswerteeinrichtung (18) programmtechnisch derart eingerichtet ist, dass die orthogonalisierten Messgrößen, die mit den gefilterten EKG-Signale auf Grundlage der Vektorkardiographie erhalten werden, in Kugelkoordinaten transformiert werden.

12. Einrichtung (10) nach Anspruch 10 oder 11, **dadurch gekennzeichnet, dass** die Sensoren (S1-S4) in einem T-Shirt (22) integriert sind, nämlich in Bereichen des T-Shirts (22), die einer korrekten Position der vier Ableitungspunkte (E1, E2, E3, E4) am Körper (14) des Patienten (11) zugeordnet sind.

13. Einrichtung (10) nach einem der Ansprüche 10 bis 12, **dadurch gekennzeichnet, dass** das KI-System (20) trainiert ist, vorzugsweise, dass das KI-System (20) mit einer Anzahl von spezifischen Lernwerten (f) trainiert ist, die auf Grundlage einer Menge (M) von Patienten mit einem bekannten Befund bestimmt worden sind, weiter vorzugsweise, dass das KI-System (20) mit einem Verfahren nach einem der Ansprüche 7 bis 13 trainiert ist, weiter vorzugsweise, dass das KI-System (20) zumindest ein neuronales Netz ($20_N$) aufweist.

**Claims**

1. A method for early detection of a presence of coronary heart disease (CHD) and /or cardiac arrhythmia (HRD) in a patient (11) to be screened, the method comprising:

   (i) non-invasive recording of ECG signals at the heart (12) of the patient (11) in the resting state,
   (ii) filtering processing of the recorded ECG signals,
   (iii) converting the filtered ECG signals into orthogonalized measurement variables based on vectorcardiography,
   **characterized by**
   (iv) inputting the orthogonalized measurement variables into a system (20) based on artificial intelligence (AI), in which already known findings data of reference patients is stored, wherein a diagnosis is made for the screened patient (11) by comparing the entered orthogonalized measurement variables with the findings data of the reference patients within the AI system,
   **and further characterized in**
   **that** the AI system (20) is trained prior to step (iv), wherein the AI system (20) comprises at least one neural network ($20_N$),
   **that** for training the AI system (20), a number of specific learning values (f) is input therein, preferably that the number of specific learning values (f) being between 10 and 30, more preferably that the number of specific learning values (f) being 20, and
   **that** the specific learning values (f) are determined by the following sequence of steps:

   (v) providing measurement variables of a set (M) of patients with a known finding, wherein these measurement variables are orthogonalized based on vectorcardiography;
   (vi) providing a plurality of time series parameters and at least one statistic;
   (vii) formring a 3D matrix, wherein the orthogonalized measurement variables of the set (M) of patients define the rows, the time series parameters define the columns, and the at least one statistical method defines the depth of this matrix;
   (viii) classifying all pairs of values of the 3D-matrix (25) according to the principle of the "Area-under-Curve" (AUC) calculation;
   (ix) selecting a pair of values from the set in step (viii) with the highest AUC value;
   (x) checking another pair of values from the set in step (viii), and selecting this pair of values, if a limit value for a correlation with the value pair of step (ix) is smaller than $1,65/\sqrt{N}$; where N = number of the data points or parameter statistics (patients) in step (vi);
   (xi) repeating step (x) for another pair of values from the set in step (viii), and selecting this pair of values if a limit value for a correlation with the previously selected value pairs is in each case smaller than $1,65/\sqrt{N}$, and
   (xii) repeating the steps (ix) to (xi) until a predetermined number of value pairs is reached, which are then defined as specific learning values (f) for training the AI system (20).

2. The method according to claim 1, wherein in step (i) the ECG signals are recorded at a total of four lead points (E1-E4) on the body (14) of the patient (11).

3. The method according to claim 2, wherein potential differentials are measured in the form of an anterior lead (A) between a first lead point (E1) and a fourth lead point (E4), a dorsal lead (D) between a second lead point (E2) and the fourth lead point (E4), a horizontal lead (H) between a third lead point (E3) and the fourth lead point (E4), a vertical lead (V) between the first lead point (E1) and the third lead point (E3), and an inferior lead (I) between the first lead point (E1) and the second lead point (E2).

4. The method according to claim 3, wherein the leads (A, D, H, I, V) between the respective lead points (E1-E4) are transformed into spherical coordinates.

5. The method according to any of claims 2 to 4, wherein, for recording the ECG signals a T-shirt (22) is used, which has four sensors (S1-S4) assigned to a correct position of the four lead points (E1-E4) on the body (14) of the patient (11).

6. The method according to any of the preceding claims, wherein in step (v) the measurement variables of the set (M) of patients (11) are provided in the form of time series, preferably in milliseconds, or in the form of heartbeats.

7. The method according to any of the preceding claims, wherein in step (vi) a plurality of statistical methods (mean value, variance, kurtosis, skew, 5% quantile, 95% quantile) are provided.

8. The method according to claim 7, wherein, after step (vii), the standardized matrix ($25_N$) is calculated from the data of the 3D- matrix (25) using a statistical method, thus achieving a uniform depth.

9. The method according to any of the preceding claims, wherein in step (viii) the AUC calculation is performed empirically or according to the principle of Johnson distribution.

10. A device for early detection of the presence of coronary heart disease (CHD) and/or cardiac arrhythmia (HRD) of a patient (11) to be screened, the device comprising:

a plurality of sensors (S1-S4) positionable at predetermined lead points (E1-E4) on the body (14) of the patient (11) so as to non-invasively record ECG signals at the heart (12) of the patient (11) in the patient's resting state; at least one filter (16) with which the recorded ECG signals can be filtered; an evaluation device (18) via which the filtered ECG signals can be converted into orthogonalized measurement variables on the basis of vectorcardiography; and a system (20) based on artificial intelligence (AI), in which already known findings data of reference patients is stored, wherein the orthogonalized measurement variables are entered into the AI system (20) and compared therein with the findings data of the reference patients in order to establish a diagnosis for the patient (11) being screened, wherein a total of four sensors (S1, S2, S3, S4) are provided corresponding to a total of four lead points (E1-E4) on the body (14) of the patient (11), wherein a position of at least the sensor (S1) assigned to the first lead point (E1) is verifiable by the device (10) and that the device (10) is programmed such that a heart-related space vector (24) is determined and displayed, wherein this space vector (24) represents the electrical field vector formed by the activity of the heart (12), wherein measurement variables of the heart (12) are recorded on the body (14) at a first lead point (E1), at a second lead point (E2), at a third lead point (E3) and at a fourth lead point (E4), wherein potential differences are measurable in the form of an anterior lead (A) between the first lead point (E1) and the fourth lead point (E4), a dorsal lead (D) between the second lead point (E2) and the fourth lead point (E4), a horizontal lead (H) between the third lead point (E3) and the fourth lead point (E4), a vertical lead (V) between the first lead point (E1) and the third lead point (E3), and an inferior lead (I) between the first lead point (E1) and the second lead point (E2), wherein an orthogonal system is be formed with the relationships:

$$x = D \cos 45° - I$$

$$y = D \sin 45° + A$$

$$z = (V - H) \sin 45°$$

wherein the measurement variables and the space vector (24) determined therefrom are mapped in this orthogonal system (x, y, z), wherein the device (10) is adapted to perform the following step sequence:

(a) performing a measurement on a patient using the first to fourth lead points (E1-E4) on the body (14) of the patient in order to obtain a cardiogram for this patient,
(b) extracting the amplitudes of the R wave from the cardiogram of step (a) for each heartbeat in the x, y and z direction,
(c) determining mean values $\mu_x$, $\mu_y$, $\mu_z$ and standard deviations $\sigma_x$, $\sigma_y$, $\sigma_z$ of the respective amplitudes recorded in millivolts from the cardiogram in step (b), wherein these mean values and standard deviations then form a calculation vector (26),
(d) forming a coefficient matrix (28) which is obtained based on a Principal Component Analysis by using different formats for measurements on reference patients,
(e) multiplying the calculation vector (26) of step (c) by the coefficient matrix of step (d) to form a resulting vector (30) with a total of six main axes ($PC_1$ - $PC_6$), wherein the coefficient matrix (28), with which in step (e) the calculation vector (26) is multiplied to form the resulting vector (30), is a 6x6 coefficient matrix,

(f) extracting the first main axis ($PC_1$) and the second main axis ($PC_2$) from the resulting vector (30) of step (e) to form a reference point ($PC_1$, $PC_2$) in the space of the first and second main axis,

(g) determining a Euclidean distance of the reference point ($PC_1$, $PC_2$) from a predetermined target point ($PC_1^{fit}$, $PC_2^{fit}$) which corresponds to a correct position of the four lead points (E1-E4) on the human body (14), and

(h) if the distance of the reference point ($PC_1$, $PC_2$) from the predetermined target point ($PC_1^{fit}$, $PC_2^{fit}$) is greater than a predetermined maximum value: performing an angular correction for a first triangle (31) formed by the first lead point (E1), the third lead point (E3) and the fourth lead point (E4), and for a second triangle (32) formed by the first lead point (E1), the second lead point (E2) and the fourth lead point (E4) so that thereby the Euclidean distance between the reference point ($PC_1$, $PC_2$) and the predetermined target point ($PC_1^{fit}$, $PC_2^{fit}$) is minimized by adapting the orthogonal system (x, y, z) to the changed geometry, wherein in step (h) the angular correction deter-mines adjustment values ($\varepsilon$, $\eta$) with which the non-orthogonal angles ($\alpha$) of the first triangle (31) and the non-orthogonal angles ($\beta$) of the second triangle (32) are corrected, so that at least an incorrect position of the first lead point (E1) on the human body (14) is compensated.

11. The device (10) according to claim 10, wherein the evaluation device (18) is programmed in such a way that the orthogonalized measurement variables obtained with the filtered ECG signals on the basis of vectorcardiography are transformed into spherical coordinates.

12. The device (10) according to claim 10 or 11, wherein the sensors (S1-S4) are integrated in a T-shirt (22), namely in areas of the T-shirt (22) which are assigned to a correct position of the four lead points (E1, E2, E3, E4) on the body (14) of the patient (11).

13. The device (10) according to any of claims 10-12, wherein the AI-system (20) is trained, preferably that the AI-system (20) is trained with a number of specific learning values (f) which are determined on the basis of a set (M) of patients with a known finding, further preferably that the the AI-system (20) is trained with a method according to any of claims 7 to 13, further preferably that the AI-system (20) comprises at least one neural network ($20_N$).

**Revendications**

1. Procédé de détection précoce de la présence d'une maladie coronarienne (MCC) et/ou d'un trouble du rythme cardiaque (TRC) chez un patient (11) à examiner, comprenant les étapes suivantes :

   (i) acquisition non invasif de signaux d'ECG au niveau du coeur (12) du patient (11) à l'état de repos,
   (ii) traitement par filtrage des signaux d'ECG enregistrés,
   (iii) transformation des signaux d'ECG filtrés en variables de mesure orthogonalisées sur la base de la vectorcardiographie,
   **caractérisé par**
   (iv) entrée des variables de mesure orthogonalisées dans un système (20) basé sur l'intelligence artificielle (IA), dans lequel sont mémorisées des données de résultats déjà connues de patients de comparaison, un diagnostic étant établi pour le patient (11) examiné par une comparaison des variables de mesure orthogonalisées entrées avec les données de résultats des patients de comparaison à l'intérieur du système IA (20),

   et **caractérisé en outre**

   **par le fait que** le système IA (20) est entraîné avant l'étape (iv), ledit système d'intelligence artificielle (20) comprenant au moins un réseau neuronal ($20_N$),
   **par le fait que**, pour l'entraînement du système IA (20), un certain nombre de valeurs d'apprentissage spécifiques (f) sont introduites dans celui-ci, de préférence en ce que le nombre de valeurs d'apprentissage spécifiques (f) est compris entre 10 et 30, de préférence encore en ce que le nombre de valeurs d'apprentissage spécifiques (f) est de 20, et
   en ce que les valeurs d'apprentissage spécifiques (f) sont déterminées par la séquence d'étapes suivante :

   (v) fournir des variables de mesure d'une quantité (M) de patients ayant un résultat connu, ces variables de mesure étant orthogonalisées sur la base de la cardiographie vectorielle,
   (vi) fournir une pluralité de paramètres de séries temporelles et au moins une statistique,

(vii) former une matrice 3D (25), les variables de mesure orthogonalisées de la quantité (M) de patients définissant les lignes, les paramètres de la série temporelle définissant les colonnes et le au moins un procédé statistique définissant la profondeur de cette matrice (25),

(viii) Classification de toutes les paires de valeurs de la matrice 3D (25) selon le principe du calcul "Area-under-Curve" (AUC),

(ix) Sélectionner une paire de valeurs dans la quantité selon l'étape (viii) avec la valeur AUC la plus élevée,

(x) vérifier une autre paire de valeurs dans l'ensemble selon l'étape (viii), et sélectionner cette paire de valeurs si une valeur seuil pour une corrélation avec la paire de valeurs de l'étape (ix) est inférieure en valeur absolue à 1,65/$\sqrt{N}$, avec N = nombre de points de données ou de statistiques de paramètres (patients) selon l'étape (vi),

(xi) répéter l'étape (x) pour une autre paire de valeurs de l'ensemble de l'étape (viii), et sélectionner cette paire de valeurs si une valeur de seuil pour une corrélation avec chacune des paires de valeurs précédemment sélectionnées est inférieure en valeur absolue à 1,65/$\sqrt{N}$, et

(xii) répéter les étapes (ix) à (xi) jusqu'à ce qu'un nombre prédéterminé de paires de valeurs soit atteint, lesquelles sont alors définies comme des valeurs d'apprentissage spécifiques (f) pour l'entraînement du système d'IA (20).

2. Procédé selon la revendication 1, **caractérisé en ce que**, à l'étape (i), les signaux d'ECG sont enregistrés à un total de quatre points de dérivation (E1-E4) sur le corps (14) du patient (11).

3. Procédé selon la revendication 2, **caractérisé en ce que** des différences de potentiel sous la forme d'une dérivée antérieure (A) entre un premier point de dérivation (E1) et un quatrième point de dérivation (E4), d'une dérivée dorsale (D) entre un deuxième point de dérivation (E2) et le quatrième point de dérivation (E4), une dérivée horizontale (H) entre un troisième point de dérivation (E3) et le quatrième point de dérivation (E4), une dérivée verticale (V) entre le premier point de dérivation (E1) et le troisième point de dérivation (E3), et une dérivée inférieure (I) entre le premier point de dérivation (E1) et le deuxième point de dérivation (E2) sont mesurées.

4. Procédé selon la revendication 3, **caractérisé en ce que** les dérivées (A, D, H, I, V) entre les points de dérivation respectifs (E1-E4) sont transformées en coordonnées sphériques.

5. Procédé selon l'une quelconque des revendications 2 à 4, **caractérisé en ce qu'**un T-shirt (22) est utilisé pour enregistrer les signaux d'ECG, ledit T-shirt comportant quatre capteurs (S1-S4) associés à une position correcte des quatre points de dérivation (E1-E4) sur le corps (14) du patient (11).

6. Procédé selon l'une quelconque des revendications précédentes, **caractérisé en ce que**, à l'étape (v), les variables de mesure de la quantité (M) de patients (11) sont mises à disposition sous forme de séries temporelles, de préférence en millisecondes ou sous forme de battements cardiaques.

7. Procédé selon l'une quelconque des revendications précédentes, **caractérisé en ce que**, à l'étape (vi), une pluralité de méthodes statistiques (valeur moyenne, variance, kurtosis, asymétrie, quantité 5 %, quantile 95 %) sont fournies.

8. Procédé selon la revendication 7, **caractérisé en ce que**, après l'étape (vii), la matrice normalisée ($25_N$) est calculée à partir des données de la matrice 3D (25) par un procédé statistique et une profondeur uniforme est ainsi obtenue.

9. Procédé selon l'une des revendications précédentes, **caractérisé en ce que**, dans l'étape (viii), le calcul de l'AUC est effectué de manière empirique ou selon le principe de la distribution de Johnson.

10. Dispositif (10) de détection précoce de la présence d'une maladie coronarienne (MCC) et/ou d'un trouble du rythme cardiaque (TRC) d'un patient (11) à examiner, comprenant une pluralité de capteurs (S1-S4) positionnables sur le corps (14) du patient (11) à examiner à des points de dérivation (E1-E4) prédéterminés, pour enregistrer ainsi de manière non invasive des signaux d'ECG au niveau du coeur (12) du patient (11), de préférence dans son état de repos,

au moins un filtre (16) avec lequel les signaux d'ECG enregistrés peuvent être filtrés,
un dispositif d'évaluation (18), avec lequel les signaux d'ECG filtrés peuvent être convertis en variables de mesure orthogonalisées sur la base de la cardiographie vectorielle,
un système (20) basé sur l'intelligence artificielle (IA), dans lequel sont mémorisées des données d'examen déjà connues de patients de comparaison, les variables de mesure orthogonalisées pouvant être introduites dans le système IA (20) et y être comparées avec les données d'examen des patients de comparaison, afin d'établir ainsi un diagnostic pour le patient (11) examiné

dans lequel un total de quatre capteurs (S1, S2, S3, S4) sont prévus, en correspondance avec un total de quatre points de dérivation (E1-E4) sur le corps (14) du patient (11), et

dans lequel une position d'au moins le capteur (S1), qui est associé au premier point de dérivation (E1), pouvant être vérifiée au moyen du dispositif (10) et le dispositif (10) étant à cet effet configuré de manière programmatique de telle sorte qu'un vecteur spatial (24) se rapportant au coeur (12) puisse être déterminé et représenté, ce vecteur spatial (24) représentant le vecteur du champ électrique qui est formé par l'activité du coeur (12), des valeurs de mesure du coeur (12) pouvant être détectées sur le corps (14) en un premier point de dérivation (E1), en un deuxième point de dérivation (E2), en un troisième point de dérivation (E3) et en un quatrième point de dérivation (E4), des différences de potentiel sous la forme d'une dérivée antérieure (A) entre le premier point de dérivation (E1) et le quatrième point de dérivation (E4), une dérivée dorsale (D) entre le deuxième point de dérivation (E2) et le quatrième point de dérivation (E4), une dérivée horizontale (H) entre le troisième point de dérivation (E3) et le quatrième point de dérivation (E4), une dérivée verticale (V) entre le premier point de dérivation (E1) et le troisième point de dérivation (E3), et une dérivée inférieure (I) entre le premier point de dérivation (E1) et le deuxième point de dérivation (E2) peuvent être mesurées, un système orthogonal étant défini par les relations suivantes:

$$x = D \cos 45° - I$$

$$y = D \sin 45° + A$$

$$z = (V - H) \sin 45°$$

et les valeurs mesurées ainsi que le vecteur spatial (24) qui en résulte sont représentés dans ce système orthogonal (x, y, z),

la séquence d'étapes suivante pouvant être exécutée par le dispositif (10):

(a) Réalisation d'une mesure sur un patient en utilisant les premier à quatrième points de dérivation (E1-E4) sur le corps (14) du patient, afin d'obtenir ainsi un cardiogramme pour ce patient,

(b) Extraction des amplitudes de l'onde R à partir du cardiogramme de l'étape (a) pour chaque battement cardiaque dans les directions x, y et z,

(c) Détermination des valeurs moyennes $\mu_x$, $\mu_y$, $\mu_z$ et des écarts types $\sigma_x$, $\sigma_y$, $\sigma_z$ des amplitudes respectivement saisies en millivolts à partir du cardiogramme selon l'étape (b), un vecteur de calcul (26) étant ensuite formé avec ces valeurs moyennes et ces écarts types,

(d) Formation d'une matrice de coefficients (28) obtenue sur la base d'une transformation d'axe principal pour des mesures sur des patients de comparaison avec différents schémas d'application,

(e) multiplier le vecteur de calcul (26) de l'étape (c) par la matrice de coefficients (28) de l'étape (d), pour former un vecteur de résultat (30) ayant un total de six axes principaux (PC1 - PC6), la matrice de coefficients (28) par laquelle le vecteur de calcul (26) est multiplié à l'étape (e) pour former le vecteur de résultat (30) étant une matrice de coefficients 6x6,

(f) Extraction du premier axe principal ($PC_1$) et du deuxième axe principal ($PC_2$) à partir du vecteur résultat (30) de l'étape (e), pour former un point de référence ($PC_1$, $PC_2$) dans l'espace du premier et du deuxième axe principal,

(g) Détermination d'une distance euclidienne du point de référence ($PC_1$, $PC_2$) par rapport à un point cible prédéterminé ($PC_1^{fit}$, $PC_2^{fit}$) correspondant à une position correcte des quatre points de dérivation (E1-E4) sur le corps humain (14), et

(h) si la distance entre le point de référence ($PC_1$, $PC_2$) et le point cible prédéterminé ($PC_1^{fit}$, $PC_2^{fit}$) est supérieure à une valeur maximale prédéterminée : effectuer une correction angulaire pour un premier triangle (31) formé par le premier point de dérivation (E1), le troisième point de dérivation (E3) et le quatrième point de dérivation (E4) et pour un deuxième triangle (32) formé par le premier point de dérivation (E1), le deuxième point de dérivation (E2) et le quatrième point de dérivation (E4), de sorte que la distance euclidienne entre le point de référence ($PC_1$, $PC_2$) et le point cible prédéterminé ($PC_1^{fit}$, $PC_2^{fit}$) est minimisée par adaption du système orthogonal (x, y, z) à la géométrie modifiée, des valeurs d'adaptation ($\varepsilon$, $\eta$) étant déterminées à l'étape (h) par la correction angulaire, avec lesquelles les angles non rectangulaires ($\alpha$) du

premier triangle (31) et les angles non rectangulaires (β) du deuxième triangle (32) sont corrigés, de sorte qu'au moins une position non correcte du premier point de dérivation (E1) sur le corps humain (14) est ainsi compensée.

**11.** Dispositif (10) selon la revendication 10, **caractérisé en ce que** le dispositif d'évaluation (18) est programmé de telle sorte que les variables de mesure orthogonalisées, qui sont obtenues avec les signaux d'ECG filtrés sur la base de la cardiographie vectorielle, sont transformées en coordonnées sphériques.

**12.** Dispositif (10) selon la revendication 10 ou 11, **caractérisé en ce que** les capteurs (S1-S4) sont intégrés dans un T-shirt (22), plus précisément dans des zones du T-shirt (22) associées à une position correcte des quatre points de dérivation (E1, E2, E3, E4) sur le corps (14) du patient (11).

**13.** Dispositif (10) selon l'une des revendications 10 à 12, **caractérisé en ce que** le système d'IA (20) est entraîné, de préférence **en ce que** le système d'IA (20) est entraîné avec un certain nombre de valeurs d'apprentissage spécifiques (f) qui ont été déterminées sur la base d'une quantité (M) de patients ayant un résultat connu, de préférence encore **en ce que** le système d'IA (20) est entraîné avec un procédé selon l'une des revendications 7 à 13, de préférence encore **en ce que** le système d'IA (20) comprend au moins un réseau neuronal ($20_N$).

Fig. 1

**Fig. 2a**

**Fig. 2b**

S4 ○ ○ S3

○ S1

22

**Fig. 3a**

S3 ○

22

**Fig. 3b**

11

14

12

X

Y

Z

Fig. 4

**Fig. 5a**

**Fig. 5b**

Fig. 6

**Fig. 7a**

**Fig. 7b**

(i)    (ii)    (iii)    (iv)

**Fig. 8**

(i)    (ii)    (iii)    (iv)

(v)    (vi)    (vii)    (viii)    (ix)    (x)    (xi)    (xii)

f

**Fig. 9**

Fig. 10a

Fig. 10b

1.  Mag-Difference-QRS_begin-R
2.  Mag-Difference-QRS_begin-QRS_end
3.  Mag-Difference-QRS_begin-T_begin
4.  Mag-Difference-QRS_begin-T
5.  Mag-Difference-QRS_begin-T_end
6.  Mag-Difference-R-QRS_end
7.  Mag-Difference-R-T_begin
8.  Mag-Difference-R-T
9.  Mag-Difference-R-T_end
10. Mag-Difference-QRS_end-T_begin
11. Mag-Difference-QRS_end-T
12. Mag-Difference-QRS_end-T_end
13. Mag-Difference-T_begin-T
14. Mag-Difference-T_begin-T_end
15. Mag-Difference-T-T_end
16. Azi-Difference-QRS_begin-R
17. Azi-Difference-QRS_begin-QRS_end
18. Azi-Difference-QRS_begin-T_begin
19. Azi-Difference-QRS_begin-T
20. Azi-Difference-QRS_begin-T_end
21. Azi-Difference-R-QRS_end
22. Azi-Difference-R-T_begin
23. Azi-Difference-R-T
24. Azi-Difference-R-T_end
25. Azi-Difference-QRS_end-T_begin
26. Azi-Difference-QRS_end-T
27. Azi-Difference-QRS_end-T_end
28. Azi-Difference-T_begin-T
29. Azi-Difference-T_begin-T_end
30. Azi-Difference-T-T_end
31. Ele-Difference-QRS_begin-R
32. Ele-Difference-QRS_begin-QRS_end
33. Ele-Difference-QRS_begin-T_begin
34. Ele-Difference-QRS_begin-T
35. Ele-Difference-QRS_begin-T_end
36. Ele-Difference-R-QRS_end
37. Ele-Difference-R-T_begin
38. Ele-Difference-R-T
39. Ele-Difference-R-T_end
40. Ele-Difference-QRS_end-T_begin
41. Ele-Difference-QRS_end-T
42. Ele-Difference-QRS_end-T_end
43. Ele-Difference-T_begin-T

44. Ele-Difference-T_begin-T_end
45. Ele-Difference-T-T_end
46. Mag-Triangle-QRS_begin-R-QRS_end
47. Mag-Triangle-QRS_begin-R-T_begin
48. Mag-Triangle-QRS_begin-R-T
49. Mag-Triangle-QRS_begin-R-T_end
50. Mag-Triangle-QRS_begin-QRS_end-T_begin
51. Mag-Triangle-QRS_begin-QRS_end-T
52. Mag-Triangle-QRS_begin-QRS_end-T_end
53. Mag-Triangle-QRS_begin-T_begin-T
54. Mag-Triangle-QRS_begin-T_begin-T_end
55. Mag-Triangle-QRS_begin-T-T_end
56. Mag-Triangle-R-QRS_end-T_begin
57. Mag-Triangle-R-QRS_end-T
58. Mag-Triangle-R-QRS_end-T_end
59. Mag-Triangle-R-T_begin-T
60. Mag-Triangle-R-T_begin-T_end
61. Mag-Triangle-R-T-T_end
62. Mag-Triangle-QRS_end-T_begin-T
63. Mag-Triangle-QRS_end-T_begin-T_end
64. Mag-Triangle-QRS_end-T-T_end
65. Mag-Triangle-T_begin-T-T_end
66. Cartesian-TimeDiff-QRS_beginX-QRS_beginY
67. Cartesian-TimeDiff-QRS_beginX-QRS_beginZ
68. Cartesian-TimeDiff-QRS_beginX-RX
69. Cartesian-TimeDiff-QRS_beginX-RY
70. Cartesian-TimeDiff-QRS_beginX-RZ
71. Cartesian-TimeDiff-QRS_beginX-QRS_endX
72. Cartesian-TimeDiff-QRS_beginX-QRS_endY
73. Cartesian-TimeDiff-QRS_beginX-QRS_endZ
74. Cartesian-TimeDiff-QRS_beginX-T_beginX
75. Cartesian-TimeDiff-QRS_beginX-T_beginY
76. Cartesian-TimeDiff-QRS_beginX-T_beginZ
77. Cartesian-TimeDiff-QRS_beginX-TX
78. Cartesian-TimeDiff-QRS_beginX-TY
79. Cartesian-TimeDiff-QRS_beginX-TZ
80. Cartesian-TimeDiff-QRS_beginX-T_endX
81. Cartesian-TimeDiff-QRS_beginX-T_endY
82. Cartesian-TimeDiff-QRS_beginX-T_endZ
83. Cartesian-TimeDiff-QRS_beginY-QRS_beginZ
84. Cartesian-TimeDiff-QRS_beginY-RX
85. Cartesian-TimeDiff-QRS_beginY-RY
86. Cartesian-TimeDiff-QRS_beginY-RZ

# Fig. 11a

87. Cartesian-TimeDiff-QRS_beginY-QRS_endX

88. Cartesian-TimeDiff-QRS_beginY-QRS_endY

89. Cartesian-TimeDiff-QRS_beginY-QRS_endZ

90. Cartesian-TimeDiff-QRS_beginY-T_beginX

91. Cartesian-TimeDiff-QRS_beginY-T_beginY

92. Cartesian-TimeDiff-QRS_beginY-T_beginZ

93. Cartesian-TimeDiff-QRS_beginY-TX

94. Cartesian-TimeDiff-QRS_beginY-TY

95. Cartesian-TimeDiff-QRS_beginY-TZ

96. Cartesian-TimeDiff-QRS_beginY-T_endX

97. Cartesian-TimeDiff-QRS_beginY-T_endY

98. Cartesian-TimeDiff-QRS_beginY-T_endZ

99. Cartesian-TimeDiff-QRS_beginZ-RX

100. Cartesian-TimeDiff-QRS_beginZ-RY

101. Cartesian-TimeDiff-QRS_beginZ-RZ

102. Cartesian-TimeDiff-QRS_beginZ-QRS_endX

103. Cartesian-TimeDiff-QRS_beginZ-QRS_endY

104. Cartesian-TimeDiff-QRS_beginZ-QRS_endZ

105. Cartesian-TimeDiff-QRS_beginZ-T_beginX

106. Cartesian-TimeDiff-QRS_beginZ-T_beginY

107. Cartesian-TimeDiff-QRS_beginZ-T_beginZ

108. Cartesian-TimeDiff-QRS_beginZ-TX

109. Cartesian-TimeDiff-QRS_beginZ-TY

110. Cartesian-TimeDiff-QRS_beginZ-TZ

111. Cartesian-TimeDiff-QRS_beginZ-T_endX

112. Cartesian-TimeDiff-QRS_beginZ-T_endY

113. Cartesian-TimeDiff-QRS_beginZ-T_endZ

114. Cartesian-TimeDiff-RX-RY

115. Cartesian-TimeDiff-RX-RZ

116. Cartesian-TimeDiff-RX-QRS_endX

117. Cartesian-TimeDiff-RX-QRS_endY

118. Cartesian-TimeDiff-RX-QRS_endZ

119. Cartesian-TimeDiff-RX-T_beginX

120. Cartesian-TimeDiff-RX-T_beginY

121. Cartesian-TimeDiff-RX-T_beginZ

122. Cartesian-TimeDiff-RX-TX

123. Cartesian-TimeDiff-RX-TY

124. Cartesian-TimeDiff-RX-TZ

125. Cartesian-TimeDiff-RX-T_endX

126. Cartesian-TimeDiff-RX-T_endY

127. Cartesian-TimeDiff-RX-T_endZ

128. Cartesian-TimeDiff-RY-RZ

129. Cartesian-TimeDiff-RY-QRS_endX

130. Cartesian-TimeDiff-RY-QRS_endY

131. Cartesian-TimeDiff-RY-QRS_endZ

132. Cartesian-TimeDiff-RY-T_beginX

133. Cartesian-TimeDiff-RY-T_beginY

134. Cartesian-TimeDiff-RY-T_beginZ

135. Cartesian-TimeDiff-RY-TX

136. Cartesian-TimeDiff-RY-TY

137. Cartesian-TimeDiff-RY-TZ

138. Cartesian-TimeDiff-RY-T_endX

139. Cartesian-TimeDiff-RY-T_endY

140. Cartesian-TimeDiff-RY-T_endZ

141. Cartesian-TimeDiff-RZ-QRS_endX

142. Cartesian-TimeDiff-RZ-QRS_endY

143. Cartesian-TimeDiff-RZ-QRS_endZ

144. Cartesian-TimeDiff-RZ-T_beginX

145. Cartesian-TimeDiff-RZ-T_beginY

146. Cartesian-TimeDiff-RZ-T_beginZ

147. Cartesian-TimeDiff-RZ-TX

148. Cartesian-TimeDiff-RZ-TY

149. Cartesian-TimeDiff-RZ-TZ

150. Cartesian-TimeDiff-RZ-T_endX

151. Cartesian-TimeDiff-RZ-T_endY

152. Cartesian-TimeDiff-RZ-T_endZ

153. Cartesian-TimeDiff-QRS_endX-QRS_endY

154. Cartesian-TimeDiff-QRS_endX-QRS_endZ

155. Cartesian-TimeDiff-QRS_endX-T_beginX

156. Cartesian-TimeDiff-QRS_endX-T_beginY

157. Cartesian-TimeDiff-QRS_endX-T_beginZ

158. Cartesian-TimeDiff-QRS_endX-TX

159. Cartesian-TimeDiff-QRS_endX-TY

160. Cartesian-TimeDiff-QRS_endX-TZ

161. Cartesian-TimeDiff-QRS_endX-T_endX

162. Cartesian-TimeDiff-QRS_endX-T_endY

163. Cartesian-TimeDiff-QRS_endX-T_endZ

164. Cartesian-TimeDiff-QRS_endY-QRS_endZ

165. Cartesian-TimeDiff-QRS_endY-T_beginX

166. Cartesian-TimeDiff-QRS_endY-T_beginY

167. Cartesian-TimeDiff-QRS_endY-T_beginZ

168. Cartesian-TimeDiff-QRS_endY-TX

169. Cartesian-TimeDiff-QRS_endY-TY

170. Cartesian-TimeDiff-QRS_endY-TZ

171. Cartesian-TimeDiff-QRS_endY-T_endX

172. Cartesian-TimeDiff-QRS_endY-T_endY

## Fig. 11b

173. Cartesian-TimeDiff-QRS_endY-T_endZ

174. Cartesian-TimeDiff-QRS_endZ-T_beginX

175. Cartesian-TimeDiff-QRS_endZ-T_beginY

176. Cartesian-TimeDiff-QRS_endZ-T_beginZ

177. Cartesian-TimeDiff-QRS_endZ-TX

178. Cartesian-TimeDiff-QRS_endZ-TY

179. Cartesian-TimeDiff-QRS_endZ-TZ

180. Cartesian-TimeDiff-QRS_endZ-T_endX

181. Cartesian-TimeDiff-QRS_endZ-T_endY

182. Cartesian-TimeDiff-QRS_endZ-T_endZ

183. Cartesian-TimeDiff-T_beginX-T_beginY

184. Cartesian-TimeDiff-T_beginX-T_beginZ

185. Cartesian-TimeDiff-T_beginX-TX

186. Cartesian-TimeDiff-T_beginX-TY

187. Cartesian-TimeDiff-T_beginX-TZ

188. Cartesian-TimeDiff-T_beginX-T_endX

189. Cartesian-TimeDiff-T_beginX-T_endY

190. Cartesian-TimeDiff-T_beginX-T_endZ

191. Cartesian-TimeDiff-T_beginY-T_beginZ

192. Cartesian-TimeDiff-T_beginY-TX

193. Cartesian-TimeDiff-T_beginY-TY

194. Cartesian-TimeDiff-T_beginY-TZ

195. Cartesian-TimeDiff-T_beginY-T_endX

196. Cartesian-TimeDiff-T_beginY-T_endY

197. Cartesian-TimeDiff-T_beginY-T_endZ

198. Cartesian-TimeDiff-T_beginZ-TX

199. Cartesian-TimeDiff-T_beginZ-TY

200. Cartesian-TimeDiff-T_beginZ-TZ

201. Cartesian-TimeDiff-T_beginZ-T_endX

202. Cartesian-TimeDiff-T_beginZ-T_endY

203. Cartesian-TimeDiff-T_beginZ-T_endZ

204. Cartesian-TimeDiff-TX-TY

205. Cartesian-TimeDiff-TX-TZ

206. Cartesian-TimeDiff-TX-T_endX

207. Cartesian-TimeDiff-TX-T_endY

208. Cartesian-TimeDiff-TX-T_endZ

209. Cartesian-TimeDiff-TY-TZ

210. Cartesian-TimeDiff-TY-T_endX

211. Cartesian-TimeDiff-TY-T_endY

212. Cartesian-TimeDiff-TY-T_endZ

213. Cartesian-TimeDiff-TZ-T_endX

214. Cartesian-TimeDiff-TZ-T_endY

215. Cartesian-TimeDiff-TZ-T_endZ

216. Cartesian-TimeDiff-T_endX-T_endY

217. Cartesian-TimeDiff-T_endX-T_endZ

218. Cartesian-TimeDiff-T_endY-T_endZ

219. areaQuotient_RT_3D

220. QRS-LoopArea

221. T-LoopArea

222. XQRS-1DIntegral

223. YQRS-1DIntegral

224. ZQRS-1DIntegral

225. XT-1DIntegral

226. YT-1DIntegral

227. ZT-1DIntegral

228. QuotientQRS_XY

229. QuotientQRS_XZ

230. QuotientQRS_YZ

231. QuotientT_XY

232. QuotientT_XZ

233. QuotientT_YZ

234. QRS-MagIntegral

235. T-MagIntegral

236. areaQuotient_RT_int

237. InterBeatTimeDiffX-QRS_beginX

238. InterBeatTimeDiffX-RX

239. InterBeatTimeDiffX-QRS_endX

240. InterBeatTimeDiffX-T_beginX

241. InterBeatTimeDiffX-TX

242. InterBeatTimeDiffX-T_endX

243. InterBeatTimeDiffY-QRS_beginY

244. InterBeatTimeDiffY-RY

245. InterBeatTimeDiffY-QRS_endY

246. InterBeatTimeDiffY-T_beginY

247. InterBeatTimeDiffY-TY

248. InterBeatTimeDiffY-T_endY

249. InterBeatTimeDiffZ-QRS_beginZ

250. InterBeatTimeDiffZ-RZ

251. InterBeatTimeDiffZ-QRS_endZ

252. InterBeatTimeDiffZ-T_beginZ

253. InterBeatTimeDiffZ-TX

254. InterBeatTimeDiffZ-T_endZ

255. Mag-Val-QRS_begin

256. Mag-Val-R

257. Mag-Val-QRS_end

258. Mag-Val-T_begin

## Fig. 11c

259. Mag-Val-T

260. Mag-Val-T_end

261. Azi-Val-QRS_begin

262. Azi-Val-R

263. Azi-Val-QRS_end

264. Azi-Val-T_begin

265. Azi-Val-T

266. Azi-Val-T_end

267. Ele-Val-QRS_begin

268. Ele-Val-R

269. Ele-Val-QRS_end

270. Ele-Val-T_begin

271. Ele-Val-T

272. Ele-Val-T_end

273. azi_RR_norm

274. azi_no_norm

275. ele_RR_norm

276. ele_no_norm

277. mag_RR_norm

278. mag_no_norm

279. muQRS

280. sigmaQRS

281. muT

282. sigmaQRS

283. superpositionT-QRS

284. sanzangle

285. age

286. weight

287. height

288. BMI

289. NaN_number

290. CrossCor_features(1)

291. CrossCor_features(2)

292. CrossCor_features(3)

# Fig. 11d

## Statistiken

1    Mittelwert

2    Varianz

3    Kurtosis

4    Schiefe

5    5% Quantil

6    95% Quantil

## Fig. 12

```
┌────────┐   ┌────────┐   ┌────────┐   ┌────────┐   ┌────────┐
│  13.1  │ → │  13.2  │ → │  13.3  │ → │  13.4  │ → │  13.5  │ → IV_T ⟹
└────────┘   └────────┘   └────────┘   └────────┘   └────────┘
```

**Fig. 13**

```
┌────────┐   ┌────────┐   ┌────────┐   ┌────────┐   ┌────────┐   ┌────────┐
│  14.1  │ → │  14.2  │ → │  14.3  │ → │  14.4  │ → │  14.5  │ → │  14.6  │ → IV_D ⟹
└────────┘   └────────┘   └────────┘   └────────┘   └────────┘   └────────┘
```

**Fig. 14**

$$
\underbrace{\begin{bmatrix} C_{11} & C_{12} & \cdots & C_{16} \\ C_{21} & C_{22} & \cdots & C_{26} \\ \vdots & & & \vdots \\ C_{61} & C_{62} & \cdots & C_{66} \end{bmatrix}}_{28} \cdot \underbrace{\begin{bmatrix} \mu_x^{\,neu} \\ \mu_y^{\,neu} \\ \mu_z^{\,neu} \\ \sigma_x^{\,neu} \\ \sigma_y^{\,neu} \\ \sigma_z^{\,neu} \end{bmatrix}}_{26} = \underbrace{\begin{bmatrix} PC1^{\,neu} \\ PC2^{\,neu} \\ PC3^{\,neu} \\ PC4^{\,neu} \\ PC5^{\,neu} \\ PC6^{\,neu} \end{bmatrix}}_{30} \Big\} \, 0
$$

## Fig. 15

Fig. 16

Fig. 17

Fig. 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 86429 B1
- WO 9936860 A
- WO 03057031 A1